Europäisches Patentamt

(19) European Patent Office

Office européen des brevets

(11) Publication number: **0 095 361**
**B1**

(12) **EUROPEAN PATENT SPECIFICATION**

(45) Date of publication of patent specification: 26.07.89

(21) Application number: 83302935.8

(22) Date of filing: 23.05.83

(51) Int. Cl.⁴: **C 12 N 15/00,** C 12 P 21/02, C 07 H 21/04 // C12R1/19

(54) Cloning vectors for expression of exogenous protein.

(30) Priority: 25.05.82 US 381992
25.05.82 US 382051

(43) Date of publication of application:
30.11.83 Bulletin 83/48

(45) Publication of the grant of the patent:
26.07.89 Bulletin 89/30

(84) Designated Contracting States:
BE CH DE FR GB IT LI LU NL SE

(56) References cited:
EP-A-0 035 384
EP-A-0 055 942
EP-A-0 075 444

CELL, vol. 18, no. 4, December 4, 1979, The Mit Press, Cambridge, Massachusetts and London; K. NAKAMURA, M. INOUYE, "DNA Sequence of the gene for the outer membrane lipoprotein of E. coli: an extremely AT-rich promoter", pp. 1109-1116

The file contains technical information submitted after the application was filed and not included in this specification

(73) Proprietor: ELI LILLY AND COMPANY
307, East McCarty Street
Indianapolis Indiana 46285 (US).

(72) Inventor: Mayne, Nancy Gail
5524A Roybury Terrace
Indianapolis Indiana 46226 (US)
Inventor: Burnett, James Paul, Jr.
7641 Brookview Lane
Indianapolis Indiana 46250 (US)
Inventor: Belegaje, Ramamoorthy
7821 Mohawk Lane
Indianapolis Indiana 46260 (US)
Inventor: Hsiung, Hansen Maxwell
108, West 88th Street
Indianapolis Indiana 46260 (US)

(74) Representative: Crowther, Terence Roger et al
Erl Wood Manor
Windlesham Surrey GU20 6PH (GB)

Courier Press, Leamington Spa, England.

(56) References cited:
**JOURNAL OF BACTERIOLOGY, vol. 146, no. 3, June 1981, Washington, DC, N. LEE et al., "Expression of the serratia marcescens lipoprotein gene in Escherichia coli", pp. 861-866**

## Description

This invention relates to novel DNA sequences and to cloning vectors (vehicles) useful in the production of protein products.

Masayori Inouye and various of his co-workers have carried out extensive studies involving gene sequences coding for outer membrane proteins of gram-negative bacteria, in particular, the lipoprotein. These investigations have demonstrated that lipoproteins are present in relatively large quantities in bacterial cells. For example, there are approximately $7.2\times10^5$ molecules of the lipoprotein of the *Escherichia coli* outer membrane per cell. Moreover, since it appears that there is only one structural gene for the lipoprotein in the *E. coli* chromosome, its transcription machinery must be highly efficient.

Recent efforts of Inoyue and associates have been directed to expression of lipoprotein using appropriately formulated plasmids in suitably transformed microorganisms and to determining and analyzing DNA sequences of various lipoprotein genes (*lpp*). Thus, in Nakamura and Inouye, *Cell 18*, 1109—1117 (1979), the DNA sequence for the outer membrane lipoprotein of *E. coli* is reported. An analysis of the promoter region of this sequence demonstrated some interesting features. First, it was noted that the segment of 261 base pairs (bp) preceding the transcription initiation site (−1 to −261) has a very high AT content (70%) in contrast to 53% for the 322 bp mRNA region, 44% for the segment of 127 bp after the transcription termination site and 49% for the average AT content of the *E. coli* chromosome. Secondly, it was noted that the first 45 bp upstream from the transcription initiation site (−1 to −45) contained 36 bases (80%) which are A or T. Thirdly, a heptanucleotide sequence analogous to the "Pribnow box" is present eight bases from the transcription initiation site. Fourthly, a sequence analogous to the "RNA polymerase recognition site" is present on both strands between positions −27 and −39. Fifthly, a long dyad symmetry is centered at the transcription initiation site.

It is postulated by Inouye and associates that these features either separately or in combination are responsible for the high degree of *lpp* promoter strength. In particular, it is postulated that the high AT content in the promoter sequence tends to destablize the helix structure of the DNA and thereby facilitates strand unwinding that is essential for initiation of transcription.

The Inouye group further has shown that a high degree of homology exists with respect to lipoprotein gene sequences of other, perhaps all, gram-negative bacteria. Thus, an analysis of the DNA sequence of the *Serratia marcescens* lipoprotein gene and comparison with that of the *E. coli lpp* gene shows a high degree of homology. [Nakamura and Inouye, *Proc Natl. Acad. Sci. USA 77*, 1369—1373 (1980)]. In particular, they showed that the promoter region is highly conserved (84% homology), having an extremely high A and T content (78%) just as in *E. coli* (80%). Moreover, the 5′ untranslated region of the lipoprotein mRNA is also highly conserved (95% homology).

More recently, in Yamagata, Nakamura, and Inouye, *J. Biol. Chem. 256*, 2194—2198 (1981), the DNA sequence of the lipoprotein gene of *Erwinia amylovora* was analyzed and compared with those of *E. coli* and *S. marcescens*. This study again confirms the high degree of homology existing in the *lpp* genes. Thus, the promoter region (−45 to −1) is highly conserved (87% relative to *E. coli* and 93% to *S. marcescens*). An extremely high A and T content (80%) exists, just as in *E. coli* (80%) and *S. marcescens* (78%). Moreover, the sequence of the untranslated region of the mRNA is highly conserved (97% relative to *E. coli* and 92% to *S. marcescens*).

The high level of constitutive transcription observed for the lipoprotein gene, based upon Inouye's studies, recommends it as a vehicle for expression of exogenous DNA fragments. Moreover, the work of Inouye *et al.* suggest that any of a wide range of lipoprotein genes of gram-negative bacteria may be so employed, including, for example, *Escherichia coli, Shigella dysenteriae, Salmonella typhimurium, Citrobacter freundii, Klebsiella aerogenes, Enterobacter aerogenes, Edwardsiella tarda, Erwinia amylovora, Serratia marcescens*, and the like.

Most recently, the suitability of the lipoprotein gene for product expression has been demonstrated by Inouye *et al.* (C. Lee, Nakamura, and Inouye, *J. Bacter. 146*, 861—866 (1981). In this work the *S. marcescens* lipoprotein gene was cloned in a lambda phage vector and then recloned in plasmid vectors pBR322 and pSC101. Both vectors carrying the *S. marcescens lpp* gene were used to transform *E. coli* cells. The evidence establishes normal expression, albeit at a level somewhat reduced relative to vectors containing the *E. coli lpp* gene. In any event, it has been established in the literature that vectors containing the *lpp* gene promoter and 5′ untranslated regions can be employed to achieve significant levels of lipoprotein expression.

By the term "vector" as used herein is meant a plasmid, phage DNA, or other DNA sequence (1) that is able to replicate in a host cell, (2) that is able to transform a host cell, and (3) that contains a marker suitable for use in identifying transformed cells.

There are two embodiments of the specific class of cloning vectors to which this invention relates. Significantly high levels of expression of exogenous protein can be achieved using either embodiment of the cloning vectors. In the first embodiment the cloning vectors are constructed to contain, in tandem, a nucleotide sequence defining the lipoprotein promoter region, a nucleotide sequence defining the lipoprotein 5′ untranslated region, and a sequence coding for an exogenous protein product, the sequence coding for such product being connected via a translation start signal codon to the 3′ terminal of the 5′

untranslated region of the lipoprotein gene. In the second embodiment the sequence coding for the exogenous protein product is connected via the aforementioned start codon and a nucleotide sequence coding for an enterokinase cleavage site to the 3′ terminal of the 5′ untranslated region of the lipoprotein gene.

European Patent Application 0055942, which falls under the terms of Article 54(3) EPC, discloses similar recombinant plasmids.

This invention relates to a recombinant DNA cloning vector useful for expressing human or bovine growth hormone, which comprises

(a) a DNA segment containing an origin of replication functional in *E. coli*;

(b) one or more DNA marker segments, each of which conveys a property useful for selection when the vector is transformed into an *E. coli* host cell; and

(c) a DNA segment comprising a sequence that defines a tandem,

(1) the promoter of an *E. coli* lipoprotein expression control sequence,

(2) the 5′ untranslated region of the *E. coli* lipoprotein expression control sequence and

(3) a translation start codon followed, without interposition of a portion or all of a nucleotide sequence coding for endogenous protein,

(i) by a seqeunce coding for human or bovine growth hormone, or

(ii) by a nucleotide sequence coding for an enterokinase cleavage site to which is joined, without interruption, a nucleotide sequence coding for human or bovine growth hormone.

The recombinant DNA cloning vector is prepared by linking DNA segments (a), (b) and (c). As noted, this invention is directed to DNA sequences and recombinant DNA cloning vectors that are highly efficient in producing exogenous protein. Each of these employs at least a portion of a lipoprotein gene (*lpp*) machinery, and, preferably, a lipoprotein gene from gram-negative bacteria. By the term "exogenous protein" as used herein is meant human and bovine growth hormone.

Examples of typical gram-negative bacteria which may serve as a source of *lpp* machinery are, for example, *Escherichia coli, Shigella dysenteriae, Salmonella typhimurium, Citrobacter freundii, Klebsiella Aerogenes, Enterobacter aerogenes, Edwardsiella tarda, Erwinia amylovora, Serratia marcescens*, and the like.

The *lpp* gene can be described in terms of five elements. In the order in which they appear in the gene, these elements are as follows: (1) the promoter region; (2) the 5′ untranslated region; (3) the lipoprotein coding sequence; (4) the 3′ untranslated region; and (5) the transcription termination site.

The function of each of these elements in gene systems is well recognized. The promoter region mediates initiation of messenger RNA (mRNA) production (transcription). The promoter may be free of external control (constitutive), under the control of a repressor, a substance that, when present, reprsses gene function, or under the control of an inducer, a substance that is required to induce gene function. The *lpp* gene is free from external control and thus is termed "constitutive".

Located at or near the promoter is the "transcription initiation site", a point at which RNA polymerase binds to initiate transcription of mRNA. Once transcription is initiated, mRNA is produced. The structure of the resulting mRNA is determined by the DNA sequences of the gene elements (2) to (4) above.

The resulting mRNA carries a sequence which is translatable into protein product. The translatable sequence is located downstream of the 5′ untranslated region and upstream of the 3′ untranslated region. Translation is mediated by binding of ribosomes to a sequence in the mRNA 5′ untranslated region denoted as the ribosome binding site and is initiated at the translation start codon (AUG) appearing as the first codon of the product gene sequence and coding as well for the amino acid methionine (Met). Translation terminates at one or more termination codons appearing at the end of the translation region.

By the techniques of recombinant DNA, it has become possible to prepare cloning vectors useful for the production of foreign (exogenous) proteins by inserting into such vectors an expression control sequence, i.e., a sequence of nucleotides that controls and regulates expression of structural genes when operatively linked to those genes. In the subject matter of this invention, the cloning vectors involve use of a portion or all of the *lpp* expression control sequence, which includes elements (1), (2), (4), and (5) are aforedescribed. Of these four elements, in the cloning vectors of this invention, only elements (1) and (2), the promoter region and the 5′ untranslated region are required.

It has been customary, using recombinant DNA methodology, to produce a foreign protein by inserting a DNA sequence coding for such foreign protein into the expression control sequence of a cloning vector at a point such that the product expressed comprises a hybrid protein. By "hybrid protein" as used herein is meant a recombinant DNA product comprising all or a portion of the natural (endogenous) protein produced by the expression control sequence (in this case, lipoprotein) to which is attached the foreign (exogenous) protein.

The properly designed hybrid protein will contain a cleavage site at the junction of the endogenous protein portion and the exogenous protein. The cleavage site permits generation of mature exogenous protein product by chemical or enzymatic treatment of the hybrid protein product.

As noted hereinbefore, it has been determined that the *lpp* expression control sequence is useful for expression of exogenous proteins. Most recently, however, it has been discovered that the *lpp* expression control sequence can be used to great advantage to express exogenous protein when the construction is designed such that a DNA segment comprising a sequence that defines, in tandem, the promoter and the 5' untranslated region of a lipoprotein expression control sequence and a translation start codon followed, without interposition of a portion or all of a nucleotide sequence coding for endogeneous protein, by a sequence coding for exogenous protein or by a nucleotide sequence coding for an enterokinase cleavage site to which is joined, without interruption, a sequence coding for exogenous protein. This is in contradistinction to a hybrid protein comprising lipoprotein or a portion thereof and exogenous protein.

In constructing the cloning vectors to which this invention relates, several elements are required. Two of the required elements are common to all useful cloning vectors. First, the vector must have a DNA segment containing a functional origin of replication (replicon). Plasmids and phage DNA by their very nature contain replicons facilitating replication in a host cell.

Secondly, the vector must have a DNA segment which conveys to a transformable host cell a property useful for selection of transformed cells from non-transformed cells. Any of a wide range of properties can be used for selection purposes. One of the most commonly used properties is antibiotic resistance, e.g., tetracycline resistance or ampicillin resistance.

The foregoing two elements generally are present in readily available and recognized cloning vectors. Examples of suitable cloning vectors are bacterial plasmids, such as plasmids from *E. coli*, including pBR322, pMB89, ColE1, pCR1; wider host range plasmids, including RP4; phage DNAs, such as lambda, and the like. Most, if not all, of the above-recognized vectors already carry the aforedescribed two elements.

A third element, specific to the vectors to which this invention relates, is the lipoprotein expression control sequence. The *E. coli* lipoprotein expression control sequence, present in plasmid pKEN111 and cultured in *E. coli* CC620, has been deposited and made a part of the stock culture collection of the Northern Regional Research Center, Agricultural Research, North Central Region, 1815 North University Street, Peoria, Illinois, 61604, from which it is available to the public under the accession number NRRL 15011. The lipoprotein expression control sequence can be removed from pKEN111 using recognized restriction sites and their corresponding restriction endonucleases. Any of a wide range of other lipoprotein expression control sequences also are available using recognized methodology. Such methods may involve, for example, preparation by synthesis or by isolation of a probe using available *lpp* sequences (e.g. pKEN111), and, taking advantage of the high degree of homology which exists between *lpp* sequences, using such probe for selecting, by hybridization, *lpp* sequences from other sources.

In producing a suitable cloning vector by insertion of the lipoprotein expression control sequence, routine methods also are used. Various sites exist within cloning vectors at which cuts can be made using a restriction endonuclease specific for such site. Any of these sites can be selected for insertion of the lipoprotein expression control sequence. As an example, in the well-recognized and documented plasmid pBR322, several suitable restriction sites exist, any of which may be employed as insertion sites. A PstI site is located within the gene for β-lactamase. Other sites outside of any specific coding region are EcoRI and PvuII. These and other sites are well recognized by those skilled in the art.

Taking advantage of any of these sites or others, insertion of a lipoprotein expression control sequence or the essential portion thereof can be readily accomplished in production of vectors defined herein.

A fourth element, again specific to the vectors to which this invention relates, is the DNA sequence coding for the exogenous protein. The key requirement with respect to the exogenous protein DNA sequence in the vectors of this invention concerns its location. It must be located downstream of the 3' end of the 5' untranslated region of the lipoprotein expression control sequence and in connection therewith via a translation start codon followed by a nucleotide sequence which codes for an enterokinase cleavage site. Necessarily, in the vectors of this invention, none of the DNA sequence coding for lipoprotein may be interposed between the 5' untranslated region and the sequence coding for exogenous protein.

A fifth element, specific to the embodiment of the vectors to which this invention relates in which the translation codon is followed by a nucleotide sequence coding for an enterokinase cleavage site to which is joined, without interruption, a sequence coding for exogenous protein. The amino acid sequence of the aforementioned cleavage site is recognized and cleaved at its carboxyl terminal by the enzyme enterokinase. The nucleotide sequence coding for an enterokinase-cleavable amino acid sequence is joined at its 5' end to the translation start codon and at its 3' end to the 5' end of the nucleotide sequence coding for the exogenous protein and is designed such that the resulting translation product comprising (methionine)-(enterokinase cleavage site)-(exogenous protein) can, by treatment with enterokinase, be cleaved with production of mature exogenous protein.

Enterokinase (3.4.21.9) has been described as "one of many hydrolases located in the brush border membrane of the intestinal duodenum". (J.J. Liepnieks and A. Light, *J. Biol. Chem. 254*, 1677—1683 (1979)). Its isolation and purification has been described in numerous publications, see, for example, Liepnieks, *supra*; S. Maroux, J. Baratti, and P. Desnuelle, *J. Biol. Chem. 246*, 5031—5039 (1971), and J Baratti, S. Maroux, D. Louvard, and P. Desnuelle, *Biochimica et Biophysica Acta 315*, 147—161 (1973).

Enterokinase appears to cleave a peptide at the carboxyl of a lysine (Lys) residue that is preceded by a multiplicity of acidic amino acids, i.e., glutamic acid (Glu) and/or aspartic acid (Asp). Thus, in A. Light, H. S. Savithri, and J. J. Liepnieks, *Anal. Biochem. 106*, 199—206 (1980), a number of amino acid sequences recognized by enterokinase are described, including many of the following:

Phe-Pro-Leu-Asp-Asp-Asp-Asp-Lys;
Val-Asp-Asp-Asp-Asp-Lys;
Phe-Pro-Ile-Glu-Glu-Asp-Lys;
Leu-Pro-Leu-Glu-Asp-Asp-Lys;
Ala-Asp-Asp-Lys;
Asp-Asp-Asp-Asp-Lys;

and the like.

The nucleotide sequences coding for any of the above as well as others can be present in the cloning vectors to which this invention relates. The only requirement is that the nucleotide sequence be one which codes for an amino acid sequence that is recognized by and, when present in a longer chain peptide, cleaved at its carboxyl terminal by enterokinase.

In construction of vectors meeting these requirements, advantage can be made of a unique Xbal restriction site that appears in the 5' untranslated region of the *E. coli* lipoprotein expression control sequence. A cut can be made at the Xbal site with removal of a portion of the 5' untranslated region. Using recognized oligonucleotide synthesis methodology, a linker can be prepared comprising the removed portion of the 5' untranslated region to which is coupled a DNA sequence coding for a start codon or the start codon followed by the enterokinase cleavage site, and a portion or all of the exogenous protein.

The DNA sequence coding for exogenous protein can be constructed synthetically, e.g., using the recognized phosphotriester method or other well-recognized methods, or its DNA sequence can be obtained by recognized methodology as a copy from isolated mRNA. Once so obtained, the cDNA copy can be cut at a restriction site located at a point as near the start codon as is available. In the first embodiment of the cloning vectors to which this invention relates a linker composed of the lipoprotein 5' untranslated region fragment removed by the Xbal cleavage followed by the cleaved portion, including start codon, of the exogenous protein, can be prepared synthetically. In the second embodiment of the cloning vectors, a linker composed of the lipoprotein 5' untranslated region fragment removed by the Xbal cleavage followed by start codon, enterokinase cleavage site, and the cleaved portion of the exogenous protein, can thus be prepared synthetically. These linkers, sufficient to bridge the gap, then are used in conjunction with remaining available elements of the lipoprotein expression control sequence to prepare a vector.

The cloning vectors to which this invention relates can be used to produce human growth hormone or bovine growth hormone. It will be recognized that the expression product of the first embodiment of the cloning vectors will contain a methionine (Met) at their amino terminal by reason of the presence of the start codon. Expression product of vectors of the second embodiment will comprise methionine (start codon), enterokinase cleavage site, and exogenous protein. Mature exogenous protein is generated by treating the latter expression product with enterokinase in accordance with recognized methiodology (see, for example, Light *et al., supra*).

Preferably, the host organism for the cloning vectors to which this invention relates is a gram-negative prokaryotic organism. Of gram-negative prokaryotic organisms, *E. coli* is especially preferred, for example, *E coli* K-12 strains, such as RV308.

Employing well recognized methodology, the cloning vectors are used to transform suitable host organisms, are amplified in such organisms, and exogenous protein product is expressed using standard fermentation conditions. The exogenous protein product is isolated by routine methods from the resulting fermentation broth.

The structure and function of cloning vectors to which this invention relates is illustrated by the examples which follow, which examples are to be read and understood in conjunction with the accompanying drawings in which:

Figures 1—4 together comprise a schematic illustration of the preparation of intermediates and starting material useful in the construction of both embodiments of the cloning vectors to which this invention relates.

Figure 5 taken in conjunction with Figures 1—4 comprises a schematic illustration of a method as described in Example 1 following for constructing a cloning vector useful for the production of methionyl human growth hormone.

Figures 6—8 together and in conjunction with Figures 1—5 comprise a schematic illustration of a method as described in Example 2 for constructing a cloning vector useful for the production of methionyl bovine growth hormone.

Figure 9, in conjunction with Figures 1—5, comprises a schematic illustration of a method as described in Example 3 for construction a cloning vector which is a variant of the cloning vector described in Example 1 and which is useful for the production of methionyl human growth hormone.

Figure 10 taken in conjunction with Figures 1—4 comprises a schematic illustration of a method as described in Example 4 for constructing a second embodiment of the cloning vectors to which this invention relates useful for the production of human growth hormone.

Figures 11—13 together and in conjunction with Figure 10 and Figures 1—4 comprise a schematic illustration of a method as described in Example 5 for constructing a cloning vector of the second embodiment for the production of bovine growth hormone.

Preparation—Intermediates and starting material common to the construction of the first and second embodiments of plasmids to which the invention relates—

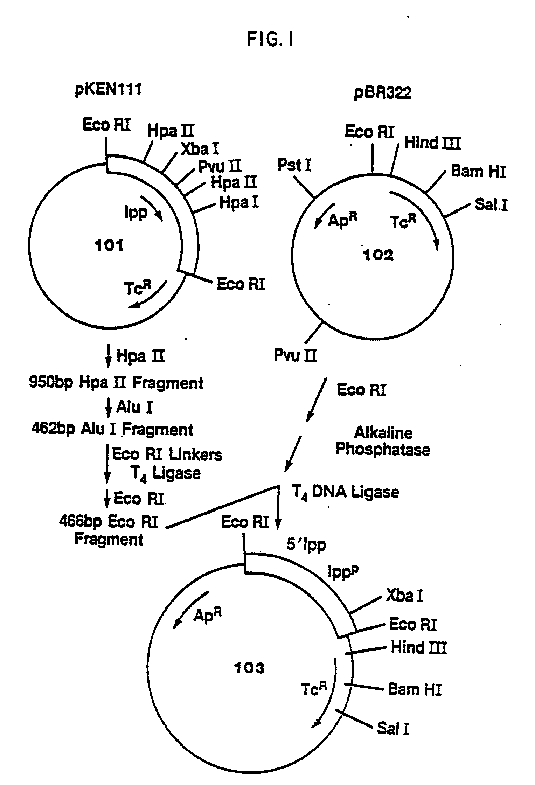

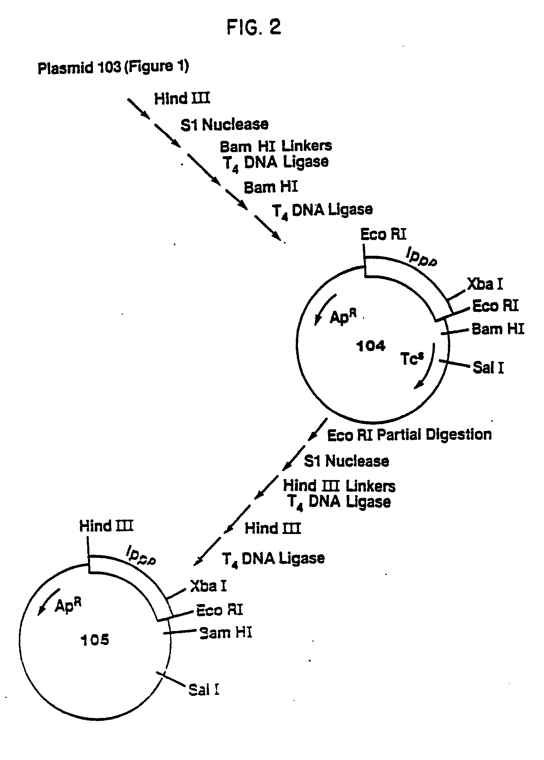

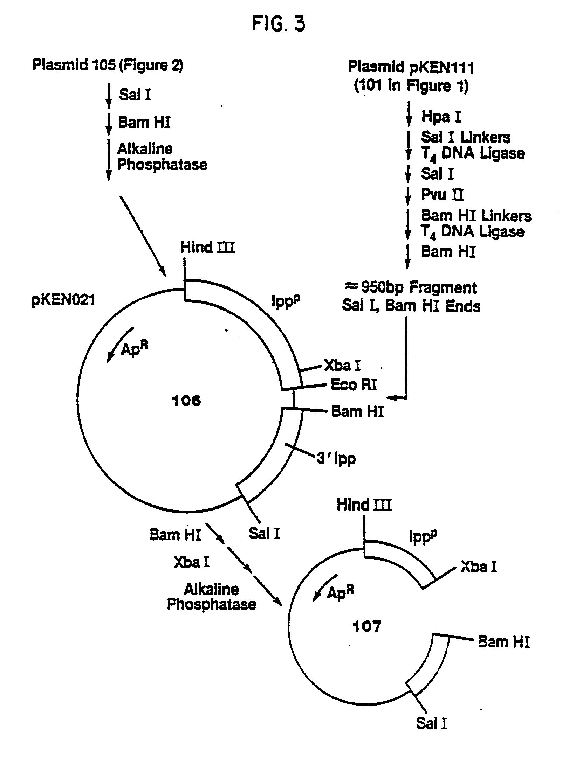

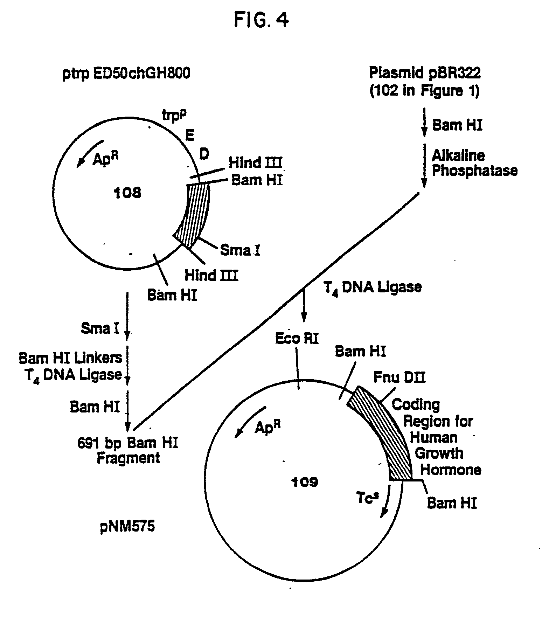

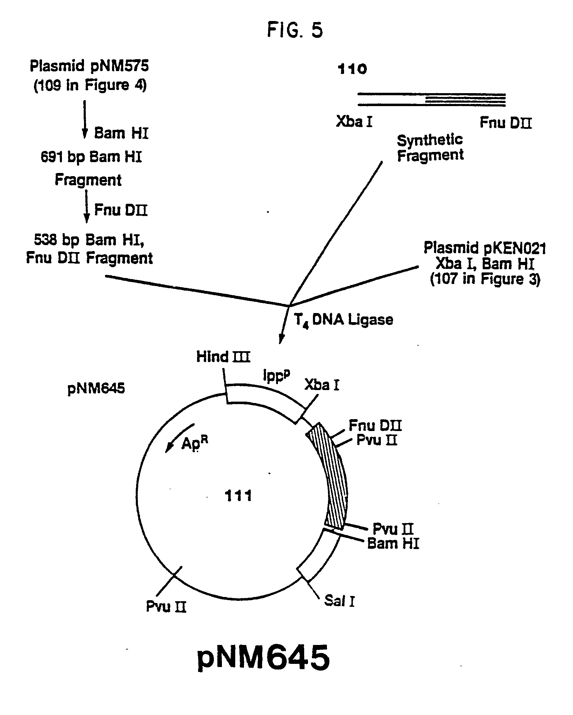

The ~5.1 kb (kilobase) fragment produced by XbaI (5'TCTAGA3'), BamHI (5'GGATCC3') cleavage of plasmid vector pKEN021 (106 in Figure 3) was used as starting material. pKEN021 is a derivative of pKEN111 (101 in Figure 1) (Lee, N., *et al., J. Bact. 146,* 861—866 (1981) and Zweibel, L. J., *et al., J. Bact. 145,* 654—656 (1981), which is on deposit in *E. coli* CC620 (NRRL Deposit No. 15011). Plasmid pKEN111 has a 2.8 kb fragment which contains the lipoprotein gene of *E. coli*. A description of this fragment is provided in Nakamura, K. and Inouye, M. *Cell 18,* 1109—1117 (1979). In pKEN021 the 650 bp (base pair) sequence between the unique EcoRI (5'GAATTC3') and SalI (5'GTCGAC3') restriction sites of pBR322 has been replaced by sequences taken from the lipoprotein gene of *E. coli*. The nucleotide sequence of all functional parts of this gene has been determined. The lipoprotein gene sequence (Nakamura, K. and Inuoye, M., *Cell 18,* 1109—1117 (1979)) includes a 462 bp AluI (5'AGCT3') fragment upstream of the first codon (methionine) of the lipoprotein gene. This fragment contains the promoter, the 5' untranslated region and the ribosome binding site. A unique XbaI (5'TCTAGA3') restriction site is located within the ribosome binding site 16 bp before the translation initiating methionine codon. A PvuII (5'CAGCTG3') restriction site located 105 bp upstream of the translation termination codon of the structural gene was changed to a BamHI (5'GGATCC3') restriction site by the addition of a synthetic DNA adapter fragment, (5'CCGGATCCGG3', obtained from Collaborative Research). The coding sequence for the last thirty-five amino acids of lipoprotein, the translation termination codon, and the sequence corresponding to the 3' untranslated region of the messenger RNA follow the BamHI site. Plasmid pKEN021 also includes some 850 bp of extraneous sequences unrelated to the lipoprotein gene and located downstream of it in the *E. coli* chromosome. These sequences were included as a consequence of the methods and restriction enzyme sites used in the original isolation of the gene.

Referring to Figures 1, 2 and 3, plasmid pKEN021 is derived from pKEN111 in the following manner: Fifty micrograms of pKEN111 (101 in Figure 1) are digested with 25 units of restriction enzyme HpaII (5'CCGG3') in 300 µl of a buffer containing 20 mM Tris:HCl pH 7.4, 10 mM MgCl$_2$, and 6 mM β-mercaptoethanol at 37°C for 2 hours. The mixture is extracted twice with 300 µl of a 50:50 mixture of phenol and chloroform, and the recovered aqueous phase is precipitated with 2.5 volumes of ethanol. The DNA pellet is dissolved in 100 µl of electrophoresis buffer and fractionated on a 5 percent polyacrylamide gel (acrylamide:bis ratio is 29:1 in all gels except where noted). The gel is stained in a solution containing 0.5 µg/ml of ethidium bromide and bands are visualized under long wave-elength ultraviolet light. A 950 bp band is isolated and recovered from the gel by electroelution into a dialysis bag. After phenol/CHCl$_3$ extraction and ethanol precipitation the recovered DNA (approximately 2.5 µg) is dissolved in 25 µl of TEN (10 mM NaCl, 10 mM Tris:HCl pH 7.4 and 1 mM sodium ethylenedinitrilotetraactate (EDTA) pH 8.0).

Two micrograms of the 950 bp HpaII fragment are digested with restriction enzyme AluI (5'AGCT3') in 200 µl of a buffer containing 50 mM NaCl, 6 mM Tris:HCl (pH 7.6), 6 mM MgCl$_2$, and 6 mM β-mercaptoethanol for 2 hours at 37°C. The DNA is fractionated on a 6 percent polyacrylamide gel, and the 462 bp AluI fragment generated is recovered and purified by the method hereinbefore described. The 462 bp AluI fragment (approximatley 1 µg) is dissolved in 10 µl of T$_4$ DNA ligase buffer (66 mM Tris:HCl pH 7.6, 10 mM MgCl$_2$, 10 mM dithiothreitol, 0.4 mM ATP) containing 150 picamoles of phosphorylated EcoRI linker (5'GGAATTCC3' from Collaborative Research) and 2 units T$_4$ DNA ligase. After incubation at 4°C. for 16 hours the mixture is heated at 65°C for 10 minutes and diluted to 100 µl with the addition of EcoRI buffer (100 mM Tris:HCl pH 7.2, 50 mM NaCl, 10 mM MgCl$_2$, 6 mM β-mercaptoethanol) and 40 units EcoRI enzyme. After 2 hours at 37°C the sample is phenol/CHCl$_3$ extracted and ethanol precipitated by the method hereinbefore described. The DNA is dissolved in 20 µl of T$_4$ DNA ligase buffer containing 0.1 unit T$_4$ DNA ligase and 0.1 µg pBR322 (102 in Figure 1) which has been linearized with EcoRI and alkaline phosphatase treated to remove end phosphates. After ligation at 4°C for 16 hours the material is used to transform a suitable *E. coli* strain (hsr⁻, hsm⁺)· such as HB101. The bacterial cells are made competent for transformation using a standard CaCl$_2$ treatment. Transformants are selected on agar plates containing 12 µg/ml of tetracycline. Plasmids are isolated from several tetracycline resistant colonies by the rapid alkaline extraction procedure described in Birnboim, H. C. and Doly, J., *Nucleic Acids Research 7,* 1513—1523 (1979). A plasmid (103 in Figure 1) containing a 466 bp XbaI, BamHI fragment (desired orientation) is selected and used as the starting material for the next step.

Two micrograms of this plasmid (103 in Figure 2) (having one HindIII (5'AAGCTT3') restriction site) are digested with 2 units of HindIII enzyme in 50 µl HindIII buffer (60 mM NaCl, 10 mM Tris:HCl pH 7.4, 10 mM MgCl$_2$ and 6 mM β-mercaptoethanol) for 1 hour at 37°C. After phenol/CHCl$_3$ extraction and ethanol precipitation the DNA is dissolved in 200 µl of a buffer containing 300 mM NaCl, 30 mM sodium acetate pH 4.25, 1 mM ZnCl$_2$ and 200 units of S1 nuclease (Miles Laboratories) which is specific for single stranded DNA. After 1 hour at 15°C the reaction is stopped by phenol/CHCl$_3$ extraction and ethanol precipitation. The plasmid, which has now had the single stranded, HindIII-generated ends removed, is dissolved in 10 µl T$_4$ DNA ligase buffer containing 20 picamoles phosphorylated BamHI linkers (5'CCGGATCCGG3', from Collaborative Research) and 2 units T$_4$ DNA ligase. After 16 hours at 4°C the reaction mixture is heated at 65°C for 10 minutes to inactivate the ligase. The mixture is diluted to 100 µl in BamHI buffer (150 mM NaCl, 20 mM Tris:HCl pH 8.0, 10 mM MgCl$_2$, 6 mM β-mercaptoethanol) containing 20 units BamHI enzyme. After 2 hours at 37°C, the mixture is purified on a 1 percent agarose gel. The gel is stained and the larger fragment (4.5 kb) is recovered by elution after freezing and purified by phenol/CHCl$_3$ extraction and ethanol precipitation. The recovered plasmid with BamHI cohesive ends is dissolved in 20 µl of T$_4$ DNA ligase buffer

containing 0.1 unit $T_4$ DNA ligase. After 16 hours at 4°C. the DNA is used to transform *E. coli* HB101. Transformants are selected by resistance to ampicillin (Ap$^r$) at 100 µg/ml and screend for sensitivity to 10 µg/ml tetracycline (Tc$^s$). Several plasmids are prepared by the previously described Birnboim procedure from colonies which are Ap$^r$Tc$^s$. These are examined for the absence of a HindIII site and the presence of a single BamHI site. EcoRI, SalI sequential digestion yields a 466 bp and a 305 bp fragment. A plasmid (104 in Figure 2) with these characteristics is selected and is modified to remove the EcoRI site located upstream of the *lpp* promoter and to convert it to a HindIII restriction site.

Two micrograms of plasmid (104 in Figure 2) are digested in 100 µl of EcoRI buffer with 0.2 units of EcoRI for 10 minutes at 37°C. The reaction is stopped by heating for 10 minutes at 65°C. After phenol/CHCl$_3$ extraction the DNA is ethanol precipitated and dissolved in 200 µl of S1 nuclease buffer containing S1 nuclease at 1000 units/ml. After 1 hour at 12°C the reaction is stopped by phenol/CHCl$_3$ extraction and ethanol precipitation. The DNA is resuspended in 10 µl of $T_4$ DNA ligase buffer containing 20 picamoles phosphorylated HindIII linker (5'CCAAGCTTGG3', from Collaborative Research) and 2 units of $T_4$ DNA ligase. After 16 hours at 4°C the ligase is inactivated by heating 10 minutes at 65°C. The reaction mixture is diluted to 150 µl in HindIII buffer containing 10 units HindIII enzyme. After incubation for 2 hours at 37°C, the mixture is fractionated on a 1 percent agarose gel. After staining in ethidium bromide, the largest band (equivalent to single cut plasmid) is recovered and purified. The plasmid is dissolved in 20 µl $T_4$ ligase buffer containing 0.2 units $T_4$ ligase, incubated 16 hours at 4°C and used to transform *E. coli* HB101. Transformants are selected for ampicillin resistance and are screened by the Birnboim procedure. Plasmid isolates are analyzed by restriction with EcoRI (1 site) and HindIII (1 site) enzymes. A plasmid (105 in Figure 2) with an EcoRI, HindIII fragment of 500 bp is selected and used as the cloning vector for addition of the 3' region of the *lpp* gene.

Two micrograms of plasmid (105 in Figure 3) are digested in 50 µl of SalI restriction buffer (150 mM NaCl, 6 mM Tris:HCl pH 7.9, 6 mM MgCl$_2$, 6 mM β-mercaptoethanol) with 2 units of SalI for 1 hour at 37°C. The reaction is diluted to 150 µl in BamHI buffer containing 2 units BamHI. After 1 hour at 37°C, 2.5 units of alkaline phosphatase are added and incubation continued for 1 hour at 65°C. The material is phenol/CHCl$_3$ extracted, ethanol precipitated, dissolved in TEN, and used as cloning vector for the *lpp* 3' fragment.

To obtain the fragment containing the *lpp* 3' region, 10 µg of pKENIII (101 in Figure 3) are digested in 200 µl of HpaI buffer (20 mM KCl, 10 mM Tris:HCl pH 7.4, 10 mM MgCl$_2$ and 6 mM β-mercaptoethanol) with 10 units of HpaI (5'GTTAAC3') for 2 hours at 37°C. After phenol/CHCl$_3$ extraction and ethanol precipitation, the DNA is dissolved in 10 µl $T_4$ DNA ligase buffer containing 20 picamoles phosphorylated SalI linker (5'GGTCGACC3', from Collaborative Research) and 2 units $T_4$ DNA ligase. After 16 hours at 4°C the ligase is inactivated by heating at 65°C for 10 minutes. The material is diluted to 100 µl in SalI buffer containing 10 units of SalI and incubated 1 hour at 37°C. The DNA is diluted to 300 µl in PvuII buffer (60 mM NaCl, 6 mM Tris:HCl, pH 7.5, 6 mM MgCl$_2$, 6 mM β-mercaptoethanol) containing 10 units PvuII restriction enzyme. After 1 hour at 37°C the DNA is fractionated on a 5 percent polyacrylamide gel. Approximately 0.5 µg of a 950 bp fragment is recovered, purified and dissolved in TEN. Two-tenths microgram of fragment is diluted into 20 µl $T_4$ DNA ligase buffer containing 20 picamoles phosphorylated BamHI linker (5'CCGGATCCGG3', from Colaborative Research) and 2 units $T_4$ DNA ligase. After 16 hours at 4°C the ligase is inactivated by heating 10 minutes at 65°C. The DNA is diluted to 100 µl in BamHI buffer containing 20 units BamHI. After 2 hours at 37°C the DNA is fractionated on a 5 percent polyacrylamide gel to remove excess linker molecules. The 950 bp fragment having BamHI and SalI cohesive ends is recovered and purified. The fragment is dissolved in 20 µl of $T_4$ DNA ligase buffer containing 0.2 µg of cloning vector described previously and 0.2 units $T_4$ DNA ligase. After incubation for 16 hours at 4°C the material is used to transform *E. coli* HB101. Plasmids are prepared from ampicillin resistant transformants and analyzed for a SalI, BamHI fragment of 950 bp. The desired plasmid (5.2 kb) is designated pKEN021 (106 in Figure 3).

Ten micrograms of pKEN021 were digested in 200 µl of XbaI/BamHI buffer (150 mM NaCl, 10 mM Tris: HCl pH 8, 10 mM MgCl$_2$, 6 mM β-mercaptoethanol) using 10 units of BamHI for 1 hour at 37°C followed by 10 units of XbaI for 1 hour at 37°C. The DNA was then treated with 2.5 units of alkaline phosphatase for 1.5 hours at 65°C, phenol/CHCl$_3$ extracted, collected by ethanol precipitation, and dissolved in 50 µl of TEN (10 mM Tris: HCl pH 7.4, 10 mM NaCl, 1 mM EDTA) for 0.2 µg/µl. This preparation (107 in Figure 3) was used as the plasmid cloning vector.

Plasmid ptrpED50chGH800 (108 in Figure 4), described in Martial J. H., *et al., Science 205,* 602—607 (1979), was used as the source of a DNA fragment containing the coding sequence for a portion of the human growth hormone gene. This fragment also is available using recognized methodology for isolating mRNA coding for human growth hormone from human pituitaries. Such methodology is described by Goodman, H. M., *et al., Methods in Enzymology 68,* 75—90 (1979). The human growth hormone gene portion of plasmid ptrpED50chGH800 contains a unique SmaI (5'CCCGGG3') restriction site 6 bp downstream from the translation termination codon of the gene. This site was changed to a BamHI site using the following procedure: 6 µg of the plasmid were digested with 6 units of SmaI in 200 µl of SmaI restriction buffer (15 mM Tris:HCl pH 8.0, 6 mM MgCl$_2$, 15 mM KCl and 6 mM β-mercaptoethanol) for 1.5 hours at 37°C. After digestion was complete, phenol/CHCl$_3$ extraction was performed, and the DNA was recovered by ethanol precipitation. The precipitated DNA was dissolved in 24 µl of TEN. Forty picamoles of phosphorylated BamHI adapter fragment (Collaborative Research) were added to 0.5 µg (0.2 picamole

ends) of the above digested plasmid in 16 µl of ligase buffer containing 2 units $T_4$ DNA ligase. Ligation was allowed to occur 2 hours at 22°C and 16 hours at 4°C. $T_4$ DNA ligase was inactivated at 65°C for 10 minutes. BamHI cohesive termini were generated by dilution into BamHI buffer containing 20 units BamHI enzyme in a final total volume of 40 µl followed by incubation at 37°C for 1 hour. The enzyme cleaved the linker sequence as well as a BamHI site located at the beginning of the cloned cDNA sequence of human growth hormone. This yielded a 691 bp fragment with cohesive BamHI ends which was separated on a 6 percent polyacrylamide gel and visualized under long wavelength ultraviolet light after staining in an ethidium bromide solution at 1 µg/ml. The gel region containing the fragment was excised and the DNA fragment was recovered by electroelution into a dialysis bag followed by ethanol precipitation. The precipitated DNA was recovered by centrifugation, dissolved in TEN, phenol/$CHCl_3$ extracted to remove ethidium bromide and ethanol precipitated. The recovered DNA fragment was ligated (using 0.2 unit $T_4$ DNA ligase in 20 µl of buffer under previously described conditions) with 0.2 µg pBR322 (102 in Figure 4) which had been cleaved at its unique BamHI site and treated with alkaline phosphatase. After 16 hours at 4°C the material was used to transform *E. coli* strain JA221 (*recA⁻, hrs⁻hsm⁺,ΔtrpE5, thr, leu, thi, lacY⁻*), which is on deposit as NRRL Deposit No. 15014. A transformation procedure as described by Wensink, P. C. *et al., Cell 3*, 315—325 (1974) was used, and transformed colonies were selected on agar plates containing 100 µg/ml ampicillin. Plasmid DNAs were isolated from sixteen of the ampicillin resistant colonies by the rapid alkaline-denaturation method previously described by Birnboim and then analyzed by restriction enzyme digestion and gel electrophoresis. Eleven of the sixteen plasmids examined were found to contain a BamHI fragment of approximately 700 bp. One of these plasmids pNM575 (109) in Figure 4) was chosen for amplification to use as a source of DNA fragment for the plasmid construction to be described. The DNA sequence of mature human growth hormone contains one FnuDII (5'CGCG3') site which is 47 bp from the first nucleotide. There are 23 recognition sites for this enzyme in pBR322. Twenty-five micrograms of pNM575 were digsted in 250 µl of BamHI buffer with 25 units of BamHI at 37°C for 1 hour. The 691 bp fragment with BamHI cohesive termini was isolated from a 6 percent polyacrylamide gel and purified by procedures described above. After purification of the fragment one third of it (equivalent to 8 µg of plasmid) was digested in 100 µl of FnuDII buffer (6 mM NaCl, 6 mM Tris:HCl pH 7.4, 6 mM $MgCl_2$, 6 mM β-mercaptoethanol) with 2.5 units FnuDII for 1.5 hours at 37°C. Electrophoresis on a 6 percent polyacrylamide gel and standard recovery procedures were used to isolate a 538 bp DNA fragment containing the coding sequence for the last 175 amino acids of the gene followed by a translation stop codon.

Example 1—Plasmid for the expression of methionyl human growth hormone using the lipoprotein promoter of *E. coli*

A. Construction

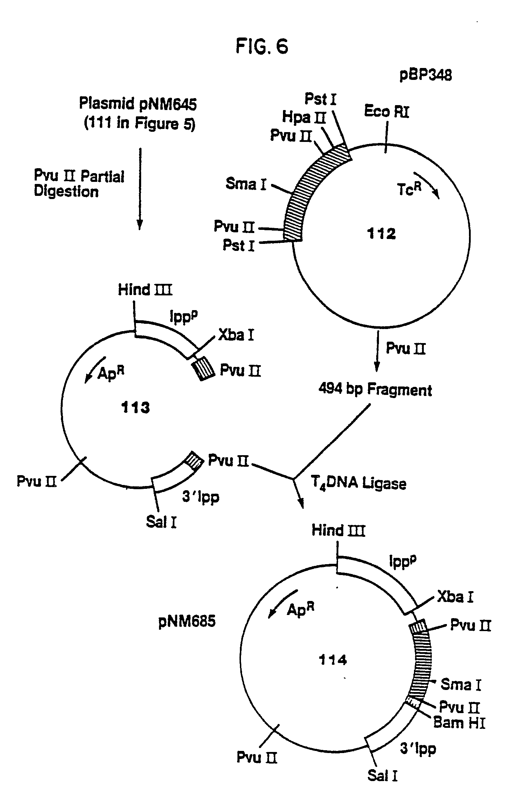

A double stranded DNA fragment (110 in Figure 5) was synthesized by the phosphotriester method to join the *lpp* promoter region with the human growth hormone coding region for direct expression of human growth hormone. The upper strand has 66 nucleotides which includes on the 5' end the 4 nucleotide single stranded sequence produced by XbaI cleavage. The lower strand has 62 nucleotides which are complementary to the last 62 nucleotides of the upper strand. The first part of the synthetic DNA fragment follows the natural sequence of the *lpp* gene from the XbaI restriction site in the ribosome binding site through the translation initiating methionine codon (19 bp) and is followed by the sequence for the first 47 nucleotides of human growth hormone to the unique FnuDII site previously described.

The double stranded DNA fragmet (110 in Figure 5) has the following structure:

```
Xbal
5' CTAGAGGGTATTAATAATGTTCCCAACCATTCCCTTATCC-
3'     TCCCATAATTATTACAAGGGTTGGTAAGGGAATAGG-

AGGCTTTTTTGACAACGCTATGCTCCG 3' Thal
TCCGAAAAAACTGTTGCGATACGAGGC 5'
```

The fragment was prepared by recognized phosphotriester methodology by which the following segments were prepared:

1) CTAGAGGGTAT
2) TAATAATGTTCC
3) CAACCATTCCC
4) TTATCCAGGC
5) TTTTTGACAACG
6) CTATGCTCCG
7) CATTATTAATACCCT
8) GGTTGGGAA
9) GGATAAGGGAAT
10) GTCAAAAAGCCT
11) CGGAGCATAGCGTT

Using the above-prepared segments, the following three duplexes were prepared.

a) Segment 1 (5'-unphosphorylated) was ligated to 5'-phosphorylated segment 2 in the presence of 5'-phosphorylated segment 7 using $T_4$ ligase to produce duplex 1 by following the established procedure [E. L. Brown. R. Belagaje, M. J. Ryan and H. G. Khorana, *Methods in Enzymology 68*, 109—151 (1979)]. The duplex was isolated by preparative gel electrophoresis on 15% polyacrylamide.

b) 5'-Phosphorylated segment 3 was ligated to 5'-phosphorylated segment 4 in the presence of 5'-phosphorylated segments 8 and 9 using $T_4$ ligase to produce duplex 2 which was isolated by preparative gel electrophoresis on 15% polyacrylamide. The reaction was performed as described above.

c) 5'-Phosphorylated segment 5 was ligated to 5'-phosphorylated segment 6 in the presence of 5'-phosphorylated segment 10 and 5'-unphosphorylated segment 11 using $T_4$ ligase as described above to produce duplex 3. The duplex was isolated by preparative gel electrophoresis on 15% polyacrylamide.

Duplexes 1, 2, and 3 then were joined by $T_4$ ligase to produce the double stranded DNA segment (110, in Figure 5) which was isolated by preparative gel electrophoresis on 15% polyacrylamide. This product was then enzymatically phosphorylated at its 5'-ends using $T_4$ polynucleotide kinase and [$\gamma$-$p^{32}$] ATP by following the established procedure.

The expression plasmid was constructed by enzymatically joining 0.1 picamole (0.4 µg) plasmid vector (107 in Figure 5), 3.2 picamoles synthetic DNA fragment (110 in Figure 5) and 0.24 picamoles (0.08 µg) of 538 bp fragment (109 in Figure 5, see Preparation) in 14 µl of ligation buffer using 2 units $T_4$ DNA ligase. After incubation for 16 hours at 4°C the mixture was used to transform *E. coli* JA221 as previously described. Transformed colonies were selected on agar plates containing 100 µg/ml ampicillin. Plasmids from 10 colonies were prepared by the previously described Birnboim screening procedure. After digestion by restriction enzymes XbaI and BamHI followed by acrylamide gel electrophoresis one plasmid was found to contain the expected 604 bp fragment. This plasmid was amplified and the DNA sequence from the XbaI site through the FnuDII site was determined by the procedure described in Maxam, A. M. and Gilbert, W., *Proc. Natl. Acad. Sci USA 74*, 560—564 (1977) and found to be correct. The plasmid is hereafter referred to as pNM645 (111 in Figure 5).

B. Expression of human growth hormone

Initial expression of human growth hormone by the plasmid pNM645 in *E. coli* JA221 was detected by modifications of the solid phase radioimmunoassay procedures described by Broome, S., and Gilbert, W., *Proc. Natl. Acad. Sci USA 75*, 2746—2749 (1978), Hitzeman, R. A., *et al., ICN-UCLA Symposia on Molecular and Cellular Biology 14*, 57—68 (1979) and Erlich, H. A., *et al., Cell 13*, 681—689 (1978).

SDS-polyacrylamide gel analysis of total bacterial cell protein performed according to Leammli, U. K., *Nature 227*, 680—685 (1970) revealed a major protein band of approximately 20,000 daltons. This band is estimated to be at least 10 percent of total protein and is not present in preparations of *E. coli* JA221 containing pKEN021. Quantitative expression was measured by a standard radioimmunoassay procedure of Twomey S. L., Beattie, J. M., and Wu, G. T., *Clin Chem 20*, 389—391 (1974) and found to be at least 2 million molecules per cell. The methionyl human growth hormone was partially purified from 500 gm *E. coli* cells by extraction with 8M urea and 1 percent Triton X100. The debris was removed by centrifugation and the supernatant containing the soluble growth hormone was fractionated on a Whatman DE52 column. The peak fractions as determined by radioimmunoassay (RIA) were pooled and subjected to isoelectric precipitation. This material was further purified on a Whatman SE53 column. The peak fractions were determined by RIA and the material was concentrated by isoelectric precipitation or ultrafiltration.

The biological activity of the recovered methionyl human growth hormone was determined by measurement of the proximal epiphyseal cartilage width in hypophysectomized female rats according to the method of Greenspan, F. S., *et al., Endocrinology 45*, 455—463 (1948). Its activity was found to be consistent with that of human growth hormone obtained from cadavers.

Example 2—Plasmid for the expression of methionyl bovine growth hormone using the lipoprotein promoter of *E. coli*

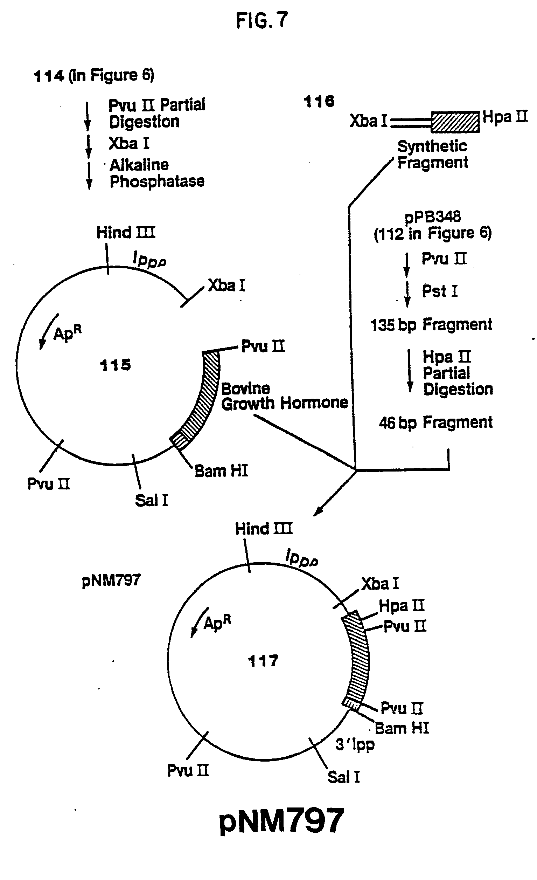

Plasmid pNM645 (111 in Figure 6), the expression plasmid for methionyl human growth hormone was used as the starting material for construction of a plasmid expressing methionyl bovine growth hormone.

Plasmid pBP348 (112 in Figure 6), described in Miller, W. L., *et al., J. Biol. Chem., 255*, 7521—7524 (1980), was used as the source of two DNA fragments containing the coding sequence for a portion of the bovine growth hormone gene. The plasmid contains an 831 bp sequence coding for bovine growth hormone cloned in the PstI (5'CTGCAG3') restriction site of pBR322. As an alternative to the method described in Miller *et al.*, the sequence for bovine growth hormone can be obtained from messenger RNA isolated from bovine pituitaries by now routine procedures described by Goodman, H. M., *et al., Methods in Enzymology 68*, 75—90 (1979).

The coding sequences for human growth hormone and bovine growth hormone are very similar and show much homology. Particularly useful in the construction of the expression plasmid for bovine growth hormone were the fragments generated by digestion with the restriction enzyme PvuII (5'CAGCTG3'). The size of the fragments produced are 497 bp in human growth hormone and 494 bp in bovine growth hormone. The corresponding restriction sites occur in the same coding frames in both sequences.

Ten micrograms of pNM645 (111 in Figure 6) containing 3 Pvull sites per molecule were digested with 1 unit of Pvull in 200 µl of Pvull restriction buffer (60 mM NaCl, 6 mM Tris:HCl pH 7.5, 6 mM MgCl₂, 6 mM β-mercaptoethanol) for 10 minutes at 37°C. The reaction was stopped by heating at 65°C for 10 minutes, and the DNA was alkaline phosphatase treated. This limited digestion procedure leads to the cleavage of one-half to two-thirds of the Pvull sites present. The fragments were separated on a one percent agarose gel and the DNA fragment (113 in Figure 6) of the size corresponding to linear plasmid with the 497 bp Pvull fragment missing (runs slightly faster than single cut plasmid) was excised, purified and used as vector in the construction of intermediate plasmid pNM685 (114 in Figure 6).

A 494 bp Pvull fragment was prepared from pBP348. Ten micrograms of the plasmid were digested in 200 µl Pvull buffer with 10 units of Pvull for 1 hour at 37°C. The fragments were separated on a 6 percent polyacrylamide gel and the 494 bp fragment (from 112 in Figure 6) was visualized and purified by methods described previously.

Intermediate plasmid pNM685 (114 in Figure 6) was constructed by ligation of 0.2 µg vector with 0.05 µg of 494 bp fragment in 20 µl of T₄ DNA ligase buffer containing 2 units T₄ DNA ligase for 16 hours at 4°C. After transformation and selection of transformants for ampicillin resistance, plasmids prepared by the previously described Birnboim procedure were analyzed for the presence of the 494 bp Pvull fragment. Proper orientation of the fragment was determined by sequential digestion with enzyme Xbal and Smal. The 494 bp Pvull fragment from the bovine growth hormone sequence has a unique asymetric Smal restriction site. Parent plasmid pNM645 contains no Smal sites. A plasmid with a 494 bp Pvull fragment and a 416 bp Xbal, Smal fragment was selected as the desired intermediate and was used in further constructions.

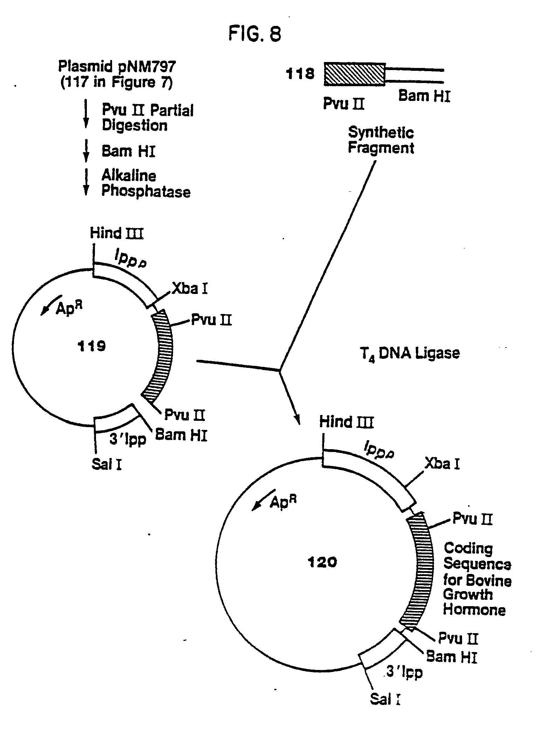

Plasmid pNM685 (114 in Figure 7) was converted to a bovine growth hormone expression plasmid by two procedures: (1) the coding sequence of the first 22 amino acids of human growth hormone was removed and replaced with the coding sequence for the first 23 amino acids of bovine growth hormone and (2) a short sequence between the second Pvull site in the coding sequence to the stop codon (which is a human growth hormone sequence) was replaced with a synthetic fragment to restore the codon for alanine, the 190th amino acid of bovine growth hormone.

Ten micrograms of pNM685 were digested with 1 unit Pvull in 200 µl Pvull buffer for 5 minutes at 37°C. The reaction was stopped by heating at 65°C for 10 minutes. The mixture of fragments was spread on a 1 percent agarose gel and linear plasmid having only a single Pvull cut per molecule was recovered and purified. This recovered material (approximately 3 µg) was digested completely with 5 units of Xbal and treated with alkaline phosphatase. The fragments were spread on a 1 percent agarose gel and the largest fragment (missing the 85 bp fragment between Xbal and the first Pvull site in human and bovine growth hormone) was recovered and used as the cloning vector (115 in Figure 7).

The DNA sequence for the first 23 amino acids (69 bp) of bovine growth hormone to the first Pvull site contains 2 restriction sites for enzyme Hpall (5'CCGG3'). The first site is 23 bp from the first nucleotide of the coding sequence. A 42 bp fragment (116 in Figure 7) corresponding to the 19 bp sequence from the Xbal site in the *lpp* ribosome binding site through the ATG initiation codon followed by the sequence for the first 23 bp of bovine growth hormone was synthesized by the phosphotriester method.

The fragment has the following structure:

```
Xbal                                                        Hpall
   5'CTAGAGGGTATTAATAATGGCTTTTCCGGCTATGTCTCTGTC3'

   3'    TCCCATAATTATTACCGAAAAGGCCGATACAGAGACAGGC    5'
```

In producing the 42 bp fragment, the following six segments were prepared:

1) CTAGAGGGTAT
2) TAATAATGGCTTTTC
3) CGGCTATGTCTCTGTC
4) CATTATTAATACCCT
5) TAGCCGGAAAAGC
6) CGGACAGAGACA

Using the above-prepared segments, 5'-phosphorylated segment 2, 5'-phosphorylated segment 3, 5'-phosphorylated segment 5 and 5'-unphosphorylated segment 6 were ligated using T₄ ligase to form a duplex, which was purified by 15% polyacrylamide gel electrophoresis. To this duplex, 5'-unphosphorylated segment 1 and 5'-phosphorylated segment 4 were added in the presence of T₄ ligase. The resulting 42 bp DNA duplex (116 in Figure 7) was isolated by 15% polyacrylamide gel electrophoresis. This duplex was then enzymatically phosphorylated at its 5'-ends using T₄ polynucleotide kinase and [γ-p³²] ATP following established procedures.

The DNA fragment of 46 bp which runs from the above described Hpall site to the Pvull site was obtained from the original pBP348 plasmid. One hundred micrograms of plasmid were digested in 400 µl of Pvull buffer with 50 units of Pvull for 2 hours at 37°C. After phenol extraction and ethanol precipitation the

EP 0 095 361 B1

DNA was dissolved in 400 µl of PstI (5'CTGCAG3') buffer (50 mM NaCl, 6 mM Tris:HCl pH 7.4, 6 mM MgCl₂, 6 mM β-mercaptoethanol) with 50 units of PstI for 2 hours at 37°C. The DNA fragments were spread on a 6 percent polyacrylamide gel (30 cm long) and the 135 bp fragment containing the desired 46 bp sequence was recovered and purified by standard procedures. One-third of the recovered DNA (equivalent to 33 µg of plasmid) was subjected to limited digestion by HpaII restriction enzyme. The DNA was digested in 100 µl HpaII buffer (20 mM Tris:HCl pH 7.4, 7 mM MgCl₂, 6 mM β-mercaptoethanol) with 1 unit of HpaII for 40 minutes at 37°C. The reaction was stopped by heating at 65°C for 10 minutes. The DNA fragments were run on a 5 percent acrylamide gel (acrylamide:bis ratio 19:1). One microgram of pBR322 digested with SauIIIA restriction enzyme was run in a separate well. This mixture of fragments contains a 46 bp fragment which is used as a size marker. The 46 bp fragment yielded by HpaII partial digestion of the 135 bp fragment (from 112 in Figure 7) was purified by standard procedures.

Two-tenths microgram plasmid vector (115 in Figure 7) having XbaI and PvuII ends was combined with 1.6 picamoles of synthetic 42 bp fragment (116 in Figure 7) and 0.5—1 picamoles 46 bp fragment (from 112 in Figure 7) in 10 µl ligation buffer with 2 units of T₄ DNA ligase and ligated for 16 hours at 4°C. The mixture was used to transform E. coli JA221, and plasmids were prepared from colonies selected by ampicillin resistance. The plasmids were screened for the presence of a 494 bp PvuII fragment and an 88 bp XbaI, PvuII fragment. Eighteen of thirty-six analyzed had these fragments. Two of the plasmids were sequenced from the XbaI site through the PvuII site and tested in a radioimmunoassay for bovine growth hormone. One was found which responded positively in the radioimmunoassay and had the correct sequence. This plasmid was designated pNM797 (117 in Figure 7). Quantitative expression was measured by standard radioimmunoassay procedures for bovine growth hormone and found to be at least $10^5$ molecules per cell.

Plasmid pNM797 (117 in Figure 8) requires one amino acid codon change for complete conversion to bovine growth hormone. This is accomplished by the removal of the 28 bp PvuII to BamHI fragment of pNM797 and replacement with a synthetic double strand fragment (13 bp upper strand, 17 bp lower strand) having the following sequence and shown at 118 in Figure 8:

$$5'\text{CTGTGCCTTCTAG}3'$$
$$3'\text{GACACGGAAGATCCTAG}5'$$

Ten micrograms of pNM797 are digested with 1 unit of PvuII in 200 µl PvuII buffer for 5 minutes at 37°C. The enzyme is inactivated by heating 10 minutes at 65°C. The sample is diluted to 300 µl with the addition of BamHI buffer and digested to completion with 10 units of BamHI for 1 hour at 37°C. This is followed by the addition of 5 units of alkaline phosphatase and incubation for 1 hour at 65°C. The DNA fragments are separated on a 1 percent agarose gel, and a DNA fragment (119 in Figure 8) the size of single cut plasmid is purified. Two-tenths microgram of this is ligated with 5 picamoles of synthetic fragment using 2 units of T₄ ligase in 20 µl ligase buffer overnight at 4°C. Following transformation and the previously described Birnboim plasmid isolation procedure, several plasmids are selected which contain the appropriate size PvuII fragment (494 bp) and XbaI, BamHI fragment (604 bp). The sequence of at least two of these is determined from the BamHI site toward the unique SmaI site and one selected with the desired sequence (120 in Figure 8).

Example 3—Variation of plasmid of Example 1

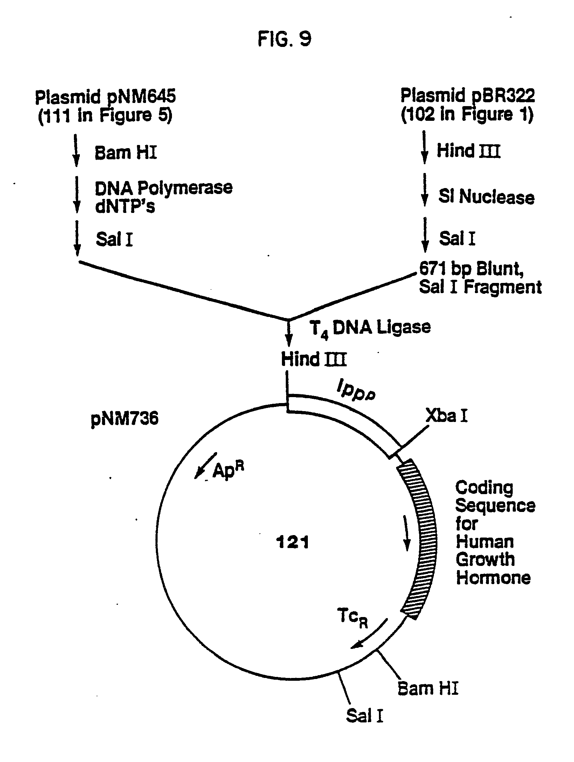

A tetracycline resistant variation of pNM645 (111 in Figure 9) was constructed by replacing the lpp 3' sequence between BamHI and SalI restriction sites with a DNA fragment derived from pBR322 (102 in Figure 9). Tetracycline resistance in pBR322 is conferred by the product of a gene whose promoter is cleaved by HindIII (5'AAGCTT3'). The coding region for the gene begins nearby and extends through the BamHI and SalI restriction sites of the plasmid. The tetracycline promoter was destroyed by digestion of the sequence with HindIII followed by S1 nuclease treatment to remove the single strand ends. Five micrograms of pBR322 were digested in 200 µl of HindIII buffer (60 mM NaCl, 20 mM Tris:HCl pH 7.4, 10 mM MgCl₂, 6 mM β-mercaptoethanol) with 10 units HindIII for 1 hour at 37°C, DNA was phenol/CHCl₃ extracted, ethanol precipitated and resuspended in 300 µl S1 buffer (300 mM NaCl, 25 mM sodium acetate pH 4.25, 1 mM ZnCl₂). S1 nuclease was added at 1000 units/ml and incubated 1 hour at 15°C. After phenol/CHCl₃ extraction and ethanol precipitation the DNA was resuspended in 200 µl SalI restriction buffer (150 mM NaCl, 6 mM Tris:HCl pH 7.9, 6 mM MgCl₂, 6 mM β-mercaptoethanol) and digested with 5 units SalI for 1 hour at 37°C. Electrophoresis on a 6 percent polyacrylamide gel was used to isolate the 617 bp fragment generated. Recovery and purification of the fragment was as described previously. Five micrograms of pNM645 were digested with 5 units of BamHI for 1 hour at 37°C. After phenol/CHCl₃ extraction and ethanol precipitation the DNA was dissolved in TEN. Two micrograms of pNM645 with BamHI cohesive termini were converted to blunt ended DNA by "filling in" using 1 unit of the large fragment of E. coli DNA polymerase I in 20 µl DNA polymerase I buffer (70 mM Tris:HCl pH 7.6, 10 mM MgCl₂, 10 mM β-mercaptoethanol, 0.5 mM each dATP, dCTP, dGTP, TTP) for 1 hour at 15°C. The enzyme was denatured at 65°C for 10 minutes and the DNA was cleaved by the addition of SalI buffer and 3 units of SalI restriction enzyme for 1 hour at 37°C. The DNA was separated on a 1 percent agarose gel and the large plasmid fragment was eluted from the gel after freezing. Ethidium bromide and agarose fragments were

12

removed by phenol/CHCl$_3$ extraction and ethanol precipitation. The plasmid was dissolved in 20 µl of TEN. Two-tengths microgram plasmid vector (0.05 picamole) was ligated with 0.2 µg 617 bp fragment (0.5 picamole) using previously described conditions. Transformed *E. coli* JA221 colonies were selected on agar plates containing 100 µg/ml ampicillin and 15 µg/ml tetracycline. Plasmids (designated pNM736, 121 in Figure 9) were isolated and found to contain desired sequence by restriction enzyme analysis. Expression of methionyl human growth hormone was found to be as high as that for pNM645.

Example 4—Plasmid for the expression of Met-Phe-Pro-Leu-Asp-Asp-Asp-Asp-Lys-Human growth hormone and its use as substrate for selective cleavage by enterokinase (3.4.21.9) to product mature human growth hormone.

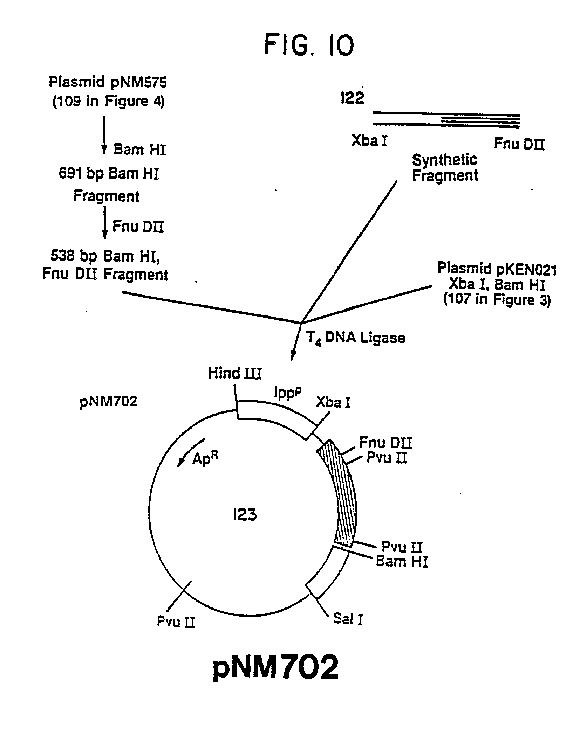

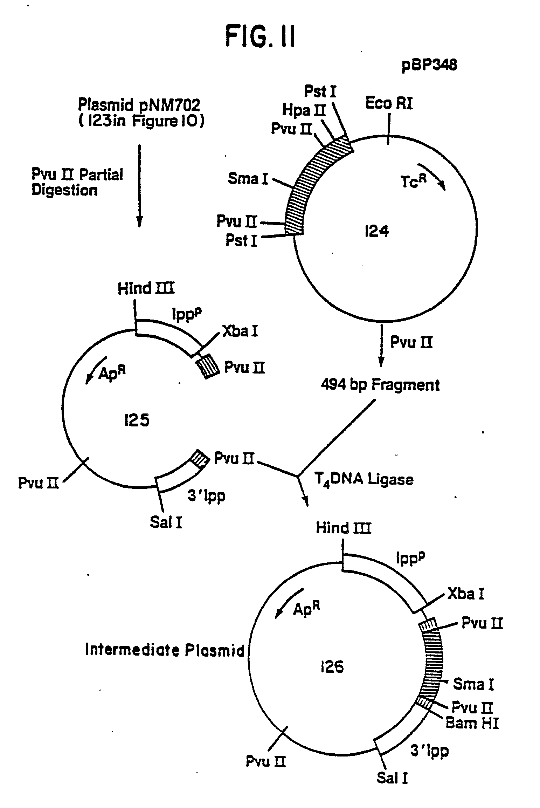

A double stranded DNA fragment (122 Figure 10) was synthesized by the phosphotriester method to join the *lpp* promoter region with the human growth hormone coding region preceded by a start codon and a coding region for a short peptide which defines a sequence recognized and cleaved by enterokinase. The upper strand has 90 nucleotides which includes on the 5' end the 4 nucleotide single stranded sequence produced by XbaI cleavage. The lower strand has 86 nucleotides which are complementary to the last 86 nucleotides of the upper strand. The first part of the synthetic DNA fragment follows the natural sequence of the *lpp* gene from the XbaI restriction site in the ribosome binding site through the translation initiating methionine codon (19 bp) and is followed by the sequence for the enterokinase cleavage site and the first 47 nucleotides of human growth hormone to the unique FnuDII site previously described.

The double stranded DNA fragment (122 in Figure 10) has the following structure:

```
      XbaI
        5'  CTAGAGGGTATTAATAATGTTCCCATTGGATGATGATGATAAGTTCCCAA-
            TCCCATAATTATTACAAGGGTAACCTACTACTACTATTCAAGGGTT-


            CCATTCCCTTATCCAGGCTTTTTGACAACGCTATGCTCCG  3'FnuDII
            GGTAAGGGAATAGGTCCGAAAAACTGTTGCGATACGAGGC  5'
```

The fragment was prepared by recognized phosphotriester methodology by which the following segments were prepared:

```
        1)   CTAGAGGGTAT
        2)   TAATAATGTTCC
        3)   CATTGGATGAT
        4)   GATGATAAGTTCC
        5)   CAACCATTCCC
        6)   TTATCCAGGC
        7)   TTTTTGACAACG
        8)   CTATGCTCCG
        9)   CATTATTAATACCCT
        10)  ATGGGAA
        11)  CTTATCATCATCCA
        12)  GGTTGGGAA
        13)  GGATAAGGGAAT
        14)  GTCAAAAAGCCT
        15)  CGGAGCATAGCGTT
```

Using the above-prepared segments, the T$_4$ ligase catalyzed joining reactions were performed stepwise as described below:

a) 5'-Unphosphorylated segment 1 was joined to 5'-phosphorylated segment 2 in the presence of 5'-phosphorylated segment 9 using T$_4$ ligase to form DNA duplex 1 [E. L. Brown, R. Belagaje, M. J. Ryan and H. G. Khorana, *Methods in Enzymology 68*, 109—151 (1979)]. The duplex was isolated by preparative gel electrophoresis on 15% polyacrylamide.

b) 5'-Phosphorylated segment 3 was joined to 5'-phosphorylated segment 4 in the presence of 5'-phosphorylated segment 11 using T$_4$ ligase to form DNA duplex 2 which was purified by 15% polyacrylamide gel electrophoresis.

c) 5'-Phosphorylated segment 5 was joined to 5'-phosphorylated segment 6 in the presence of 5'-phosphorylated segments 12 and 13 using T$_4$ ligase to form DNA duplex 3 which was purified by 15% polyacrylamide gel electrophoresis.

d) 5'-Phosphorylated segment 7 was joined to 5'-phosphorylated segment 8 in the presence of 5'-phosphorylated segment 14 and 5'-unphosphorylated segment 15 using T$_4$ ligase to form DNA duplex 4 which was purified by 15% polyacrylamide gel electrophoresis.

e) The DNA duplexes 2, 3 and 4 then were joined together by T$_4$ ligase to form DNA duplex 5 which was purified by 15% polyacrylamide gel electrophoresis.

f) To the DNA duplex 1 then were added 5'-phosphorylated segment 10 and DNA duplex 5 in the

presence of $T_4$ ligase, and the resulting DNA dupulex (110 in Figure 10) was purified by 10% polyacrylamide gel electrophoresis. This DNA duplex then was enzymatically phosphorylated using $T_4$ polynucleotide kinase and [γ-p$^{32}$] ATP by following the established procedure.

The expression plasmid was constructed by enzymatically joining 0.1 picamole (0.4 μg) plasmid vector (107 in Figure 5), 0.025 picamoles synthetic DNA fragment (110 in Figure 5) and 0.3 picamoles (0.08 μg) of 538 bp fragment (109 in Figure 10, see Preparation) in 24 μl of ligation buffer using 1.5 units $T_4$ DNA ligase. After incubation for 16 hours at 4°C the mixture was used to transform E. coli JA221 as previously described. Transformed colonies were selected on agar plates containing 100 μg/ml ampicillin. Plasmids from 19 colonies were prepared by the prevoiusly described Birnboim screening procedure. After digestion by restriction enzymes XbaI and BamHI followed by acrylamide gel electrophoresis 12 plasmids were found to contain the expected 628 bp fragment.

Eight of the positive plasmids were digested sequentially with XbaI and PvuII and seven of these yielded a 109 bp fragment. The sequence of one plasmid was determined by the procedure described by Maxam, A. M. and Gilbert, W. *Proc. Natl. Sci. USA 74,* 560—564 (1977) and found to be correct. The plasmid was designated pNM702 (123 in Figure 10). Expression of human growth hormone was detected by a standard radio-immunoassay procedure described by Twomey, S. L., et al., *Clin. Chem. 20,* 389—391 (1974). Quantitative expression was determined to be at least 2 million molecules per cell.

Met-phe-pro-leu (asp)$_4$ lys-human growth hormone was partially purified from 500 gm E. coli cells by extraction with 8M urea and 1 percent Triton X100. The debris was removed by centrifugation and the supernatant containing the soluble human growth hormone product was fractionated on a Whatman DE52 column. The peak fractions as determined by radioimmunoassay (RIA) were pooled and subjected to isoelectric precipitation. This material was further purified on a Whatman SE53 column. The peak fractions were determined by RIA and the material was concentrated by isoelectric precipitation or ultrafiltration.

The partially purified material was subjected to cleavage by enterokinase. Crude porcine intestine enterokinase (Miles Laboratories) was further purified by the method of Anderson, et al., *Biochemistry 16,* 3354—3360 (1977). Enterokinase was incubated with substrate, and samples were removed at intervals for examination on an isoelectric focusing gel. The starting material has an isoelectric point of 4.3 and can be seen to shift with time to a band having the isoelectric point of human growth hormone (4.91).

Example 5—Plasmid for the expression of Met-Phe-Pro-Leu-Asp-Asp-Asp-Asp-Lys-Bovine growth hormone using the lipoprotein promoter of E. coli

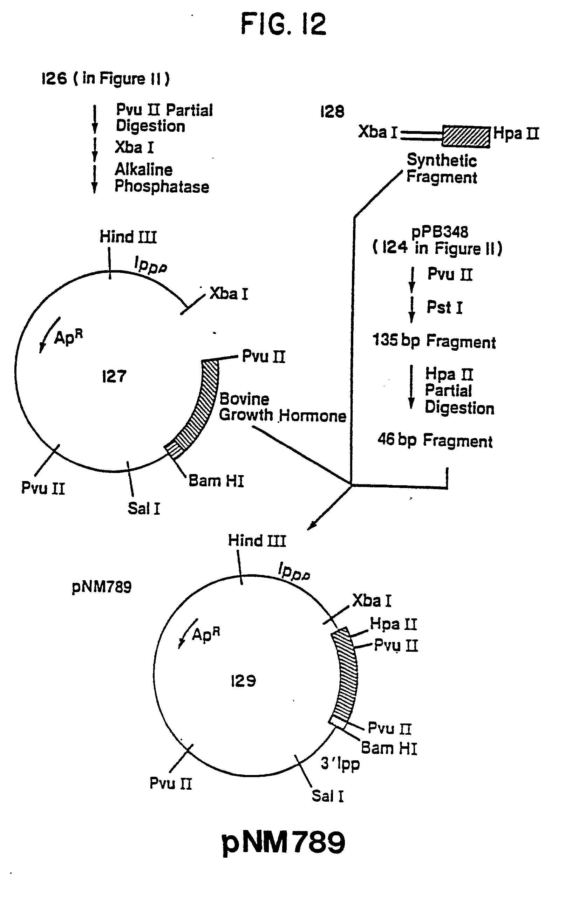

Plasmid pNM702 (123 in Figure 11), the expression plasmid for human growth hormone was used as the starting material for construction of a plasmid expressing Met-Phe-Pro-Leu-Asp-Asp-Asp-Asp-Lys-bovine growth hormone.

Plasmid pBP348 (124 in Figure 11), described in Miller, W. L., et al., *J. Biol. Chem. 255,* 7521—7524 (1980), was used as the source of two DNA fragments containing the coding sequence for a portion of the bovine growth hormone gene. The plasmid contains an 831 bp sequence coding for bovine growth hormone cloned in the PstI (5'CTGCAG3') restriction site of pBR322. As an alternative to the method described in Miller et al., the sequence for bovine growth hormone can be obtained from messenger RNA isolated from bovine pituitaries by now routine procedures described by Goodman H. M., et al., *Methods in Enzymology 68,* 75—90 (1979).

As noted above, the coding sequences for human growth hormone and bovine growth hormone are very similar and show much homology. The fragments generated by digestion with the restriction enzyme PvuII (5'CAGCTG3') were also useful in the construction of this expression plasmid for bovine growth hormone.

Ten micrograms of pNM702 (123 in Figure 11) containing 3 PvuII sites per molecule are digested with 1 unit of PvuII in 200 μl of PvuII restriction buffer (60 mM NaCl, 6 mM Tris:HCl pH 7.5, 6 mM MgCl$_2$, 6 mM β-mercaptoethanol) for 10 minutes at 37°C. The reaction is stopped by heating at 65°C for 10 minutes, and the DNA was alkaline phosphatase treated. This limited digestion procedure leads to the cleavage of one-half to two-thirds of the PvuII sites present. The fragments are separated on a one percent agarose gel and the DNA fragment (125 in Figure 11) of the size corresponding to linear plasmid with the 497 bp PvuII fragment missing (runs slightly faster than single cut plasmid) was excised, purified and used as vector in the construction of intermediate plasmid (126 in Figure 11).

A 494 bp PvuII fragment was prepared from pBP348. Ten micrograms of the plasmid were digested in 200 μl PvuII buffer with 10 units of PvuII for 1 hour at 37°C. The fragments were separated on a 6 percent polyacrylamide gel and the 494 bp fragment (from 124 in Figure 11) was visualized and purified by methods described previously.

Intermediate plasmid (126 in Figure 11) is constructed by ligation of 0.2 μg vector with 0.05 μg of 494 bp fragments in 20 μl of $T_4$ DNA ligase buffer containing 2 units $T_4$ DNA ligase for 16 hours at 4°C. After transformation and selection of transformants for ampicillin resistance, plasmids prepared by the previously described Birnboim procedure are analyzed for the presence of the 494 bp PvuII fragment. Proper orientation of the fragment is determined by sequential digestion with enzymes XbaI and SmaI. The 494 bp PvuII fragment from the bovine growth hormone sequence has a unique asymetric SmaI restriction site. Parent plasmid pNM702 contains no SmaI sites. A plasmid with a 494 bp PvuII fragment and a 440 bp XbaI, SmaI fragment is selected as the desired intermediate and is used in further constructions.

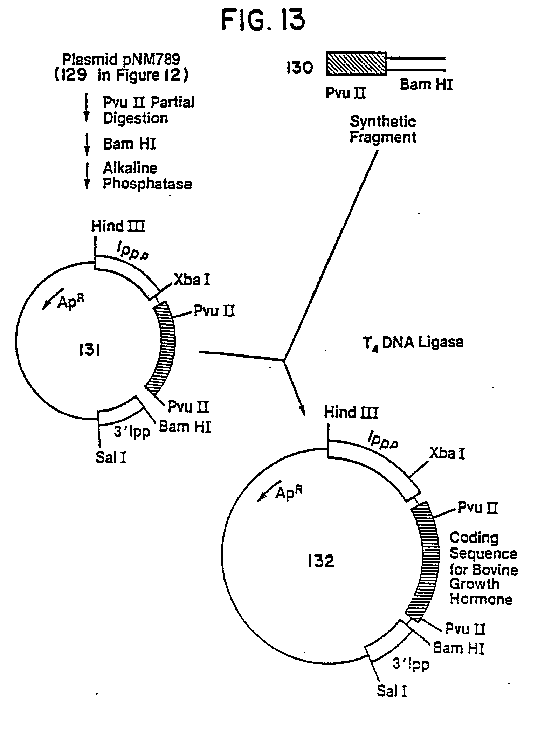

Intermediate plasmid (126 in Figure 12) is converted to the desired fused bovine growth hormone expression plasmid by two procedures: (1) the coding sequence of the first 30 amino acids of enterokinase substrate-human growth hormone was removed and replaced with the coding sequence for the first 31 amino acids of enterokinase substrate-bovine growth hormone and (2) a short sequence between the second PvuII site in the coding sequence to the stop codon (which is a human growth hormone sequence) is replaced with a synthetic fragment to restore the codon for alanine, the 190th amino acid of bovine growth hormone.

Ten micrograms of the intermediate plasmid (126 in Figure 12) are digested with 1 unit PvuII in 200 µl PvuII buffer for 5 minutes at 37°C. The reaction is stopped by heating at 65°C for 10 minutes. The mixture of fragments is spread on a 1 percent agarose gel and linear plasmid having only a single PvuII cut per molecule is recovered and purified. This recovered material (approximately 3 µg) is digested completely with 5 units of XbaI and treated with alkaline phosphatase. The fragments are spread on a 1 percent agarose gel and the largest fragment (missing the 109 bp fragment between XbaI and the first PvuII site in human and bovine growth hormone) is recovered and used as the cloning vector (127 in Figure 12).

The DNA sequence for the first 23 amino acids (69 bp) of bovine growth hormone to the first PvuII site contains 2 restriction sites for enzyme HpaII (5'CCGG3'). The first site is 23 bp from the first nucleotide of the coding sequence. A 63 bp fragment (128 in Figure 12) was synthesized by the phosphotriester method. This fragment corresponds to the 19 bp sequence from the XbaI site in the *lpp* ribosome binding site through the ATG initiation codon followed by the coding sequence for Phe-Pro-Leu-Asp-Asp-Asp-Asp-Lys (24 bp) and 20 nucleotides of the coding sequence of bovine growth hormone from Phe to the first HpaII site.

The fragment has the following structure:

```
Xbal
     5'   CTAGAGGGTATTAATAATGTTCCCATTGGATGATGATGATAAG-
     3'       TCCCATAATTATTACAAGGGTAACCTACTACTACTATTC-

                                        Hpall
     TTCCCAGCCATGTCCTTGTC         3'
     AAGGGTCGGTACAGGAACAGGC       5'
```

In producing the 63 bp fragment, the following nine segments were prepared:

1) CTAGAGGGTAT
2) TAATAATGTTCC
3) CATTGGATGAT
4) GATGATAAGTTCC
5) CAGCCATGTCCTTGTC
6) ATGGGAACATTATTAATACCCT
7) TTATCATCATCATCCA
8) ATGGCTGGGAAC
9) CGGACAAGGAC

Using the above-prepared segments, the T$_4$ ligase catalyzed joining reactions were performed stepwise as described below:

a) 5'-Unphosphorylated segment 1 was joined to 5'-phosphorylated segment 2 in the presence of 5'-phosphorylated segment 6 using T$_4$ ligase to form DNA duplex 1 which was purified by 15% polyacrylamide gel electrophoresis.

b) 5'-Phosphorylated segments 3, 4 and 5 were joined in the presence of 5'-phosphorylated segments 7 and 8 and 5'-unphosphorylated segment 9 using T$_4$ ligase to form DNA duplex 2 which was purified by 15% polyacrylamide gel electrophoresis.

c) Duplexes 1 and 2 then were joined by T$_4$ ligase to form DNA duplex (128 in Figure 12) which was purified by 15% polyacrylamide gel electrophoresis. This DNA duplex was then enzymatically phosphorylated using T$_4$ polynucleotide kinase and [γ-p$^{32}$]ATP following established procedure.

The DNA fragment of 46 bp which runs from the above described HpaII site to the PvuII site was obtained from the original pBP348 plasmid. One hundred micrograms of plasmid were digested in 400 µl of PvuII buffer with 50 units of PvuII for 2 hours at 37°C. After phenol extraction and ethanol precipitation the DNA was dissolved in 400 µl of PstI (5'CTGCAG3') buffer (50 mM NaCl, 6 mM Tris:HCl pH 7.4, 6 mM MgCl$_2$, 6 mM β-mercaptoethanol) with 50 units of PstI for 2 hours at 37°C. The DNA fragments were spread on a 6 percent polyacrylamide gel (30 cm long) and the 135 bp fragment containing the desired 46 bp sequence was recovered and purified by standard procedures. One-third of the recovered DNA (equivalent to 33 µg of plasmid) was subjected to limited digestion by HpaII restriction enzyme. The DNA was digested in 100 µl HpaII buffer (20 mM Tris:HCl pH 7.4, 7 mM MgCl$_2$, 6 mM β-mercaptoethanol) with 1 unit of HpaII for 40 minutes at 37°C. The reaction was stopped by heating at 65°C for 10 minutes. The DNA fragments were run on a 5 percent acrylamide gel (acrylamide:bis ratio 19:1). One microgram of pBR322 digested with SauIIIA restriction enzyme was run in a separate well. This mixture of fragments contains a 46bp fragment which is used as a size marker. The 46 bp fragment yielded by HpaII partial digestion of the 135 bp fragment (from 124 in Figure 12) was purified by standard procedures.

Two-tenths microgram plasmid vector (127 in Figure 12) having XbaI and PvuII ends was combined with 3.2 picamoles of synthetic 63 bp fragment (128 in Figure 12) and 0.5—1 picamoles 46 bp fragment (from 124 in Figure 12) in 10 µl ligation buffer with 2 units of $T_4$ DNA ligase and ligated for 16 hours at 4°C. The mixture was used to transform E. coli JA221, and plasmids were prepared from colonies selected by ampicillin resistance. The plasmids were screened for the presence of a 494 bp PvuII fragment and a 109 bp XbaI, PvuII fragment. One of twelve analyzed had these fragments. This plasmid was sequenced from the XbaI site through the PvuII site and tested in a radioimmunoassay for bovine growth hormone. It was found to respond positively in the radioimmunoassay and had the correct sequence. This plasmid was designated pNM789 (129 in Figure 12). Quantitative expression was measured by standard radioimmunoassay procedures for bovine growth hormone and found to be at least $10^5$ molecules per cell.

Plasmid pNM789 (129 in Figure 13) requires one amino acid codon change for complete conversion to bovine growth hormone. This is accomplished by the removal of the 28 bp PvuII to BamHI fragment of pNM789 and replacement with a synthetic double strand fragment (13 bp upper strand, 17 bp lower strand) having the following sequence and shown at 130 in Figure 13:

$$5'\text{CTGTGCCTTCTAG}3'$$
$$3'\text{GACACGGAAGATCCTAG}5'$$

Ten micrograms of pNM789 are digested with 1 unit of PvuII in 200 µl PvuII buffer for 5 minutes at 37°C. The enzyme is inactivated by heating 10 minutes at 65°C. The sample is diluted to 300 µl with the addition of BamHI buffer and digested to completion with 10 units of BamHI for 1 hour at 37°C. This is followed by the addition of 5 units of alkaline phosphatase and incubation for 1 hour at 65°C. The DNA fragments are separated on a 1 percent agarose gel, and a DNA fragment (131 in Figure 13) the size of single cut plasmid is purified. Two-tenths microgram of this is ligated with 5 picamoles of synthetic fragments using 2 units of $T_4$ ligase in 20 µl ligase buffer overnight at 4°C. Following transformation and the previously described Birnboim plasmid isolation procedure, several plasmids are selected which contain the appropriate size PvuII fragment (494 bp) and XbaI, BamHI fragment (628 bp). The sequence of at least two of these is determined from the BamHI site toward the unique SmaI site and one selected with the desired sequence (132 in Figure 13).

## Claims

1. A recombinant DNA cloning vector useful for expressing human or bovine growth hormone, which comprises
   (a) a DNA segment containing an origin of replication functional in E. coli;
   (b) one or more DNA marker segments, each of which conveys a property useful for selection when the vector is transformed into an E. coli host cell; and
   (c) a DNA segment comprising a sequence that defines in tandem,
   (1) the promoter of an E. coli lipoprotein expression control sequence,
   (2) the 5' untranslated region of the E. coli lipoprotein expression control sequence and
   (3) a translation start codon followed, without interposition of a portion or all of a nucleotide sequence coding for endogenous protein,
   (i) by a sequence coding for human or bovine growth hormone, or
   (ii) by a nucleotide sequence coding for an enterokinase cleavage site to which is joined, without interruption, a nucleotide sequence coding for human or bovine growth hormone.

2. The vector of Claim 1 which contains in whole or in part the 3' untranslated region of the E. coli lipoprotein expression control sequence, said 3' untranslated region being located downstream of the sequence coding for the human or bovine growth hormone.

3. The vector of Claim 1 or 2 which contains in whole or in part the transcription termination region of E. coli lipoprotein expression control sequence, said transcription termination region being located downstream of the sequence coding for the human or bovine growth hormone.

4. The vector of Claim 3 which contains in whole or in part the transcription termination region of E. coli lipoprotein expression control sequence, said transcription termination region being located downstream of the 3' untranslated region.

5. The vector of any of Claims 1 to 4 wherein the translation start codon is followed by a sequence coding for human growth hormone.

6. The vector of any of Claims 1 to 4 wherein the translation start codon is followed by a sequence coding for bovine growth hormone.

7. The vector of Claim 5 which is the plasmid shown in Figure 5 named pNM645 (number 111).

8. The vector of Claim 6 which is the plasmid shown in Figure 7 named pNM797 (number 117).

9. The vector of any of Claims 1 to 4 wherein the translation start codon is followed by a nucleotide sequence coding for an enterokinase cleavage site to which is joined, without interruption, a nucleotide seqeuence coding for human growth hormone.

10. The vector of any of Claims 1 to 4 wherein the translation start codon is followed by a nucleotide sequence coding for an enterokinase cleavage site to which is joined, without interruption, a nucleotide sequence coding for bovine growth hormone.

11. The vector of Claim 9 or 10 wherein the enterokinase cleavage site codes for a sequence of amino acids comprising Asp-Asp-Asp-Asp-Lys.

12. The vector of Claim 11 wherein the DNA sequence coding for the enterokinase cleavage site comprises

GATGATGATGATAAG
CTACTACTACTATTC.

13. The vector of Claim 9 or 10 wherein the enterokinase cleavage site codes for Phe-Pro-Leu-Asp-Asp-Asp-Asp-Lys.

14. The vector of Claim 13 wherein the DNA sequence coding for the enterokinase cleavage site is

TTCCCATTGGATGATGATGATAAG
AAGGGTAACCTACTACTACTATTC.

15. The vector of Claim 14 which is the plasmid shown in Figure 10 named pNM702 (number 123).

16. The vector of Claim 14 which is the plasmid shown in Figure 12 named pNM789 (number 129).

17. The vector of Claim 14 which as shown in Figure 13 is obtained by removal of the 28 bp PvuII to BamHI fragment of pNM789 and replacement with a synthetic double strand fragment (fragment 130) having the following sequence:

$5'$CTGTGCCTTCTAG$3'$
$3'$GACACGGAAGATCCTAG$5'$

18. A polypeptide having the formula, proceeding from the N-terminal to the C-terminal end, of: Met-Phe-Pro-Leu-Asp-Asp-Asp-Asp-Lys-R wherein R represents the amino acid sequence of human growth hormone.

19. A polypeptide having the formula, proceeding from the N-terminal to the C-terminal end, of: Met-Phe-Pro-Leu-Asp-Asp-Asp-Asp-Lys-R wherein R represents the amino acid sequence of bovine growth hormone.

**Patentansprüche**

1. Für die Expression von Human- oder Rinder-Wachstumshormon brauchbarer rekombinanter DNS-Klonierungsvektor, der folgende Bestandteil umfaßt:

(a) ein DNS-Segment, das einen in E. coli funktionalen Replikationsursprung enthält;

(b) ein oder mehrere DNS-Marker-Segment(e), von denen jedes eine für die Selektion nach Transformation des Vektors in eine E. coli-Wirtszelle brauchbare Eigenschaft vermittelt; und

(c) ein DNS-Segment, das eine Sequenz umfaßt, die nacheinander angeordnet definiert

(1) den Promoter eines Kontrollsequenz für die E. coli-Lipoprotein-Expression,

(2) den nicht translatierten 5'-Bereich der Kontrollsequenz für die E. coli-Lipoprotein-Expression, und

(3) ein Translations-Startkodon, ohne Zwischenschaltung eines Teils oder einer gesamten für ein endogenes Protein kodierenden Nucleotid-Sequenz gefolgt von

(a) einer für Human- oder Rinder-Wachstumshormon kodierenden Sequenz oder

(b) einer Nucleotid-Sequenz, die für eine Enterokinase-Spaltstelle kodiert, an die ohne Unterbrechung eine Nucleotid-Sequenz gebunden ist, die für Human- oder Rinder-Wachstumshormon kodiert.

2. Vektor nach Anspruch 1, der als Ganzes oder teilweise den nicht translatieren 3'-Bereich der Kontrollsequenz für die E. coli-Lipoprotein-Expression enthält, wobei der nicht translatierte 3'-Bereich im Strang nach der Sequenz angeordnet ist, die für das Human- oder Rinder-Wachstumshormon kodiert.

3. Vektor nach Anspruch 1 oder 2, der als Ganzes oder teilweise den Terminationsbereich der Transkription der Kontroll-Sequenz der E. coli-Lipoprotein-Expression enthält, wobei der Terminationsbereich der Transkription im Strang nach der Sequenz angeordnet ist, die für das Human- oder Rinder-Wachstumshormon kodiert.

4. Vektor nach Anspruch 3, der als Ganzes oder teilweise den Terminationsbereich der Transkription der Kontroll-Sequenz der E. coli-Lipoprotein-Expression enthält, wobei der Terminationsbereich der Transkription im Strang nach dem nicht translatierten 3'-Bereich angeordnet ist.

5. Vektor nach einem der Ansprüche 1 bis 4, worin dem Start-Kodon für die Translation eine Sequenz folgt, die für Human-Wachstumshormon kodiert.

6. Vektor nach einem der Ansprüche 1 bis 4, worin dem Start-Kodon für die Translation eine Sequenz folgt, die für Rinder-Wachstumshormon kodiert.

17

7. Vektor nach Anspruch 5, nämlich das in Figur 5 gezeigte Plasmid mit dem Namen pNM645 (Nummer 111).

8. Vektor nach Anspruch 6, nämlich das in Figur 7 geziegte Plasmid mit dem Namen pNM797 (Nummer 117).

9. Vektor nach einem der Ansprüche 1 bis 4, worin dem Start-Kodon für die Translation eine Nucleotid-Sequenz folgt, die für eine Enterokinase-Spaltstelle kodiert, an die ohne Unterbrechung eine Nucleotid-Sequenz gebunden ist, die für Human-Wachstumshormon kodiert.

10. Vektor nach einem der Ansprüche 1 bis 4, worin dem Start-Kodon für die Translation eine Nucleotid-Sequenz folgt, die für eine Enterokinase-Spaltstelle kodiert, an die ohne Unterbrechung eine Nucleotid-Sequenz gebunden ist, die für Rinder-Wachstumshormon kodiert.

11. Vektor nach Anspruch 9 oder 10, worin die Enterokinase-Spaltstelle für eine Asp-Asp-Asp-Asp-Lys umfassende Sequenz von Aminosäuren kodiert.

12. Vektor nach Anspruch 11, worin die für die Enterokinase-Spaltstelle kodierende DNS-Sequenz die Sequenz

GATGATGATGATAAG
CTACTACTACTATTC

umfaßt.

13. Vektor nach Anspruch 9 oder 10, worin die Enterokinase-Spaltstelle für Phe-Pro-Leu-Asp-Asp-Asp-Asp-Lys kodiert.

14. Vektor nach Anspruch 13, worin die für die Enterokinase-Spaltstelle kodierende DNS-Sequenz die Sequenz

TTCCCATTGGATGATGATGATAAG
AAGGGTAACCTACTACTACTATTC

ist.

15. Vektor nach Anspruch 14, nämlich das in Figur 10 gezeigte Plasmid mit dem Namen pNM702 (Nummer 123).

16. Vektor nach Anspruch 14, nämlich das in Figur 12 gezeigte Plasmid mit dem Namen pNM789 (Nummer 129).

17. Vektor nach Anspruch 14, der—wie in Figure 13 gezeigt—erhalten wird durch Entfernen des 28 BP umfassenden und von Pvu II bis Bam HI reichenden Fragments aus pNM789 und Ersetzen durch ein synthetisches doppelsträngiges Fragment (Fragment 130) mit der Sequenz

$5'$CTGTGCCTTCTAG$3'$
$3'$GACACGGAAGATCCTAG$5'$.

18. Polypeptid mit der von N-terminalen Ende in Richtung des C-terminalen Endes angegebenen Formel Met-Phe-Pro-Leu-Asp-Asp-Asp-Asp-Lys-R, worin R für die Aminosäure-Sequenz des Human-Wachstumshormons steht.

19. Polypeptid mit der von N-terminalen Ende in Richtung des C-terminalen Endes angegebenen Formel Met-Phe-Pro-Leu-Asp-Asp-Asp-Asp-Lys-R, worin R für die Aminosäure-Sequenz des Rinder-Wachstumshormons steht.

**Revendications**

1. Vecteur de clonage d'ADN recombinant, utile pour l'expression de l'hormone de croissance humaine ou bovine, ce vecteur comprenant
   (a) un segment d'ADN contenant une origine de réplication, celle-ci étant fonctionnelle dans *E. coli;*
   (b) un segment marqueur d'ADN ou plus, chacun de ceux-ci conférant une propriété utile à des fins de sélection, lorsque le vecteur est transformé en une cellule-hôte de *E. coli;* et
   (c) un segment d'ADN comprenant une séquence qui définit un tandem,
   (1) le promoteur d'une séquence de contrôle d'expression de lipoprotéine de *E. coli,*
   (2) la région 5' non traduite de la séquence de contrôle de l'expression de lipoprotéine de *E. coli,* et
   (3) un codon de départ de traduction, ce codon étant suivi, sans qu'une partie ou la totalité d'un codage de séquence nucléotidique pour une protéine endogène vienne s'interposer,
   (i) d'un codage de séquence pour l'hormone de croissance humaine ou bovine, ou
   (ii) d'un codage de séquence nucléotidique pour un site de clivage d'entérokinase, auquel est adjoint, sand interruption, un codage de séquence nucléotidique pour l'hormone de croissance humaine ou bovine.

2. Vecteur selon la revendication 1, ce vecteur contenant la totalité ou une partie de la région 3' non traduite de la séquence de contrôle d'expression de lipoprotéine de *E. coli,* cette région 3' non traduite étant située en aval du codage de séquence pour les hormones de croissance humaine ou bovine.

3. Vecteur selon la revendication 1 ou 2, ce vecteur contenant la totalité ou une partie de la région de terminaison de transcription de la séquence de contrôle d'expression de lipoprotéine de *E. coli,* cette région

18

de terminaison de transcription étant située en aval du codage de séquence pour les hormones de croissance humaine ou bovine.

4. Vecteur selon la revendication 3, ce vecteur contenant la totalité ou une partie de la région de terminaison de transcription de la séquence de contrôle de l'expression de lipoprotéine de *E. coli*, cette région de terminaison de transcription étant située en aval de la région 3' non traduite.

5. Vecteur selon l'une quelconque des revendications 1 à 4, dans lequel le codon de départ de traduction est suivi d'un codage de séquence pour l'hormone de croissance humaine.

6. Vecteur selon l'une quelconque des revendications 1 à 4, dans lequel le codon de départ de traduction est suivi d'un codage de séquence pour l'hormone de croissance bovine.

7. Vecteur selon la revendication 5, ce vecteur étant le plasmide représenté en figure 5 et dénommé pNM645/(n° 111).

8. Vecteur selon la revendication 6, ce vecteur étant le plasmide représenté en figure 7 et dénommé pNM797/(n° 117).

9. Vecteur selon l'une quelconque des revendications 1 à 4, dans lequel le codon de départ de traduction est suivi d'un codage de séquence nucléotidique pour un site de clivage d'entérokinase, auquel est joint, sans interruption, un codage de séquence nucléotidique pour l'hormone de croissance humaine.

10. Vecteur selon l'une quelconque des revendications 1 à 4, dans lequel le codon de départ de traduction est suivi d'un codage de séquence nucléotidique pour un site de clivage d'entérokinase, auquel est joint, sans interruption un codage de séquence nucléotidique pour l'hormone de croissance bovine.

11. Vecteur selon la revendication 9 ou 10, dans lequel le site de clivage d'entérokinase code une séquence d'acides aminés, comprenant Asp-Asp-Asp-Asp-Lys.

12. Vecteur selon la revendication 11, dans lequel le codage de séquence d'ADN pour le site de clivage d'entérokinase comprend

GATGATGATGATAAG
CTACTACTACTATTC.

13. Vecteur selon la revendication 9 ou 10, dans lequel le site de clivage d'entérokinase code la séquence

Phe-Pro-Leu-Asp-Asp-Asp-Asp-Lys.

14. Vecteur selon la revendication 13, dans lequel le codage de séquence d'ADN pour le site de clivage d'entérokinase est

TTCCCATTGGATGATGATGATAAG
AAGGGTAACCTACTACTACTATTC.

15. Vecteur selon la revendication 14, ce vecteur étant le plasmide illustré en figure 10 et dénommé pNM702/(n° 123).

16. Vecteur selon la revendication 14, ce vecteur étant le plasmide illustré en figure 12 et dénommé pNM789/(n°129).

17. Vecteur selon la revendication 14 qui, comme illustré en figure 13, est obtenu par élimination du fragment Pvull de 28 pb jusqu'au fragment BamHI de pNM789 et par son remplacement par un fragment bicaténaire synthétique (fragment 130), comportant la séquence suivante:

$$5'CTGTGCCTTCTAG^{3'}$$
$$_{3'}GACACGGAAGATCCTAG_{5'}$$

18. Polypeptide, depuis la terminaison N jusqu'à la terminaison C, répondant à la formule: Met-Phe-Pro-Leu-Asp-Asp-Asp-Asp-Lys-R, où R représente la séquence d'acides aminés de l'hormone de croissance humaine.

19. Polypeptide, depuis la terminaison N jusqu'à la terminaison C, répondant à la formule: Met-Phe-Pro-Leu-Asp-Asp-Asp-Asp-Lys-R, où R représente la séquence d'acides aminés de l'hormone de croissance bovine.

## FIG. I

pKEN111

Eco RI  Hpa II
Xba I
Pvu II
Hpa II
Hpa I

Ipp

101

TcR          Eco RI

↓ Hpa II

950bp Hpa II Fragment

↓ Alu I

462bp Alu I Fragment

Eco RI Linkers
↓ T₄ Ligase

↓ Eco RI

466bp Eco RI
Fragment

pBR322

Eco RI
Hind III
Pst I
Bam HI
ApR     TcR
Sal I
102

Pvu II

Eco RI

Alkaline
Phosphatase

T₄ DNA Ligase

Eco RI

5'Ipp

IppP
Xba I
Eco RI
Hind III

ApR

103

Bam HI

TcR

Sal I

# FIG. 2

Plasmid 103 (Figure 1)

Hind III

S1 Nuclease

Bam HI Linkers
T$_4$ DNA Ligase

Bam HI

T$_4$ DNA Ligase

Eco RI Partial Digestion

S1 Nuclease

Hind III Linkers
T$_4$ DNA Ligase

Hind III

T$_4$ DNA Ligase

## FIG. 3

Plasmid 105 (Figure 2)

↓ Sal I

↓ Bam HI

↓ Alkaline
  Phosphatase

Plasmid pKEN111
(101 in Figure 1)

↓ Hpa I

↓ Sal I Linkers
  T₄ DNA Ligase

↓ Sal I

↓ Pvu II

↓ Bam HI Linkers
  T₄ DNA Ligase

↓ Bam HI

≈ 950bp Fragment
Sal I, Bam HI Ends

pKEN021

Hind III

Ipp$^p$

Ap$^R$

106

Xba I
Eco RI

Bam HI

3' Ipp

Bam HI

Sal I

Xba I

Alkaline
Phosphatase

Hind III

Ipp$^p$

Xba I

Ap$^R$

107

Bam HI

Sal I

3

## FIG. 4

## FIG. 5

Plasmid pNM575
(109 in Figure 4)

↓ Bam HI

691 bp Bam HI
Fragment

↓ Fnu DII

538 bp Bam HI,
Fnu DII Fragment

**110**

Xba I                    Fnu DII
Synthetic
Fragment

Plasmid pKEN021
Xba I, Bam HI
(107 in Figure 3)

T₄ DNA Ligase

pNM645

Hind III
lpp^p   Xba I

Ap^R

111

Fnu DII
Pvu II

Pvu II
Bam HI

Pvu II                    Sal I

# pNM645

## FIG. 6

## FIG. 7

**114 (in Figure 6)**

↓ Pvu II Partial Digestion
↓ Xba I
↓ Alkaline Phosphatase

**116**

Xba I ▭▨▨▨ Hpa II
Synthetic Fragment

pPB348
(112 in Figure 6)

↓ Pvu II
↓ Pst I

135 bp Fragment

↓ Hpa II Partial Digestion

46 bp Fragment

Hind III
Ipp*p*

Xba I

Ap^R

**115**

Pvu II

Bovine Growth Hormone

Pvu II
Sal I
Bam HI

Hind III
Ipp*p*

pNM797

Ap^R

**117**

Xba I
Hpa II
Pvu II

Pvu II
Bam HI
3'Ipp
Pvu II
Sal I

# pNM797

## FIG. 8

Plasmid pNM797
(117 in Figure 7)

Pvu II Partial Digestion

Bam HI

Alkaline Phosphatase

118

Pvu II    Bam HI

Synthetic Fragment

Hind III

Ipp<sub>p</sub>

Xba I

Pvu II

Ap<sup>R</sup>

119

Pvu II

3'Ipp    Bam HI

Sal I

T₄ DNA Ligase

Hind III

Ipp<sub>p</sub>

Xba I

Pvu II

Ap<sup>R</sup>

120

Coding Sequence for Bovine Growth Hormone

Pvu II

Bam HI

3'Ipp

Sal I

## FIG. 9

Plasmid pNM645
(111 in Figure 5)

Plasmid pBR322
(102 in Figure 1)

↓ Bam HI

↓ Hind III

↓ DNA Polymerase
dNTP's

↓ Sl Nuclease

↓ Sal I

↓ Sal I

671 bp Blunt,
Sal I Fragment

↓ T₄ DNA Ligase

Hind III

Ippₚ

pNM736

Xba I

ApR

121

Coding
Sequence
for
Human
Growth
Hormone

TcR

Bam HI

Sal I

# FIG. 10

Plasmid pNM575
(109 in Figure 4)

↓ Bam HI

691 bp Bam HI
Fragment

↓ Fnu DII

538 bp Bam HI,
Fnu DII Fragment

122

Xba I                    Fnu DII
Synthetic
Fragment

Plasmid pKEN021
Xba I, Bam HI
(107 in Figure 3)

T₄ DNA Ligase

pNM702

Hind III
Ipp$^P$    Xba I

Ap$^R$

123

Fnu DII
Pvu II

Pvu II
Bam HI

Pvu II          Sal I

**pNM702**

# FIG. II

pBP348

Plasmid pNM702
( 123in Figure 10)

Pvu II Partial
Digestion

Pst I
Hpa II
Pvu II
Sma I
Pvu II
Pst I
Eco RI
$Tc^R$
124

Pvu II

494 bp Fragment

Hind III
Ipp$^p$
Xba I
Pvu II
$Ap^R$
125
Pvu II
3'Ipp
Sal I
Pvu II

$T_4$DNA Ligase

Hind III
Ipp$^p$
$Ap^R$
Intermediate Plasmid
126
Xba I
Pvu II
Sma I
Pvu II
Bam HI
3'Ipp
Pvu II
Sal I

# FIG. 12

126 ( in Figure 11 )

Pvu II Partial Digestion

Xba I

Alkaline Phosphatase

128

Xba I ▭▨▨ Hpa II

Synthetic Fragment

Hind III

lppₚ

Xba I

Apᴿ

127

Pvu II

Sal I

Pvu II

Bovine Growth Hormone

Bam HI

pPB348 ( 124 in Figure 11 )

Pvu II

Pst I

135 bp Fragment

Hpa II Partial Digestion

46 bp Fragment

pNM789

Hind III

lppₚ

Apᴿ

129

Pvu II

Xba I

Hpa II

Pvu II

Pvu II

Bam HI

3′ lpp

Sal I

## pNM789

# FIG. 13

Plasmid pNM789
(129 in Figure 12)

↓ Pvu II Partial Digestion

↓ Bam HI

↓ Alkaline Phosphatase

130

Pvu II    Bam HI

Synthetic Fragment

T₄ DNA Ligase

Hind III

Ippₚ

ApR

131

Xba I

Pvu II

Pvu II

3'Ipp    Bam HI

Sal I

Hind III

Ippₚ

ApR

132

Xba I

Pvu II

Coding Sequence for Bovine Growth Hormone

Pvu II
Bam HI

3'Ipp

Sal I